# EUROPÄISCHE PATENTSCHRIFT

(11) **EP 2 137 154 B1**
(45) Veröffentlichungstag und Bekanntmachung des Hinweises auf die Patenterteilung: **15.07.2015**
(21) Anmeldenummer: 08734934.6
(22) Anmeldetag: 01.04.2008
(51) Int. Cl.: C07D 213/16, C07D 405/04, C09K 19/04, C09K 19/34

(54) **PYRIDIN-VERBINDUNGEN FÜR FLÜSSIGKRISTALLINE MISCHUNGEN**
PYRIDINE COMPOUNDS FOR LIQUID-CRYSTALLINE MIXTURES
COMPOSÉS DE PYRIDINE POUR MÉLANGES À CRISTAUX LIQUIDES

(30) Priorität: 24.04.2007 DE 102007019670
(43) Veröffentlichungstag der Anmeldung: 30.12.2009
(73) Patentinhaber: Merck Patent GmbH, 64293 Darmstadt (DE)
(72) Erfinder: LIETZAU, Lars, 64295 Darmstadt (DE); CZANTA, Markus, 64289 Darmstadt (DE)
(86) Internationale Anmeldenummer: PCT/EP2008/002582
(87) Internationale Veröffentlichungsnummer: WO 2008/128622

(56) Entgegenhaltungen:
- EP-A- 0 786 445
- EP-A- 1 900 792
- WO-A-2005/019378
- WO-A-2008/019743
- DE-A1- 19 949 333
- US-A- 5 445 763
- BREMER M: "LIQUID CRYSTALS BASED ON 2-FLUOROPYRIMIDINE AND -PYRIDINE: SYNTHESIS, DIELECTRIC ANISOTROPY AND PHASE BEHAVIOR" ADVANCED MATERIALS, WILEY VCH, WEINHEIM, DE, Bd. 7, Nr. 9, 1. September 1995 (1995-09-01), Seiten 803-807, XP000520468 ISSN: 0935-9648
- PAVLUCHENKO A I ET AL: "LIQUID CRYSTALLINE 2,5-DISUBSTITUTED PYRIDINE DERIVATIVES" 1. Dezember 1995 (1995-12-01), LIQUID CRYSTALS, TAYLOR AND FRANCIS, ABINGDON, GB, PAGE(S) 811 - 821 , XP000554522 ISSN: 0267-8292 Verbindungen 1-11, 4-10 - 4-13

## Beschreibung

Die Erfindung betrifft 2,5-substituierte Pyridin-Derivate und 3-Fluoropyridin-Derivate mit einer Difluormethylenoxy-Gruppe sowie ihre Verwendung als Komponente(n) in flüssigkristallinen Medien. Darüber hinaus betrifft die vorliegende Erfindung Flüssigkristall- und elektrooptische Anzeigeelemente, welche die erfindungsgemäßen, flüssigkristallinen Medien enthalten. Die erfindungsgemäßen Verbindungen weisen eine Difluormethylenoxy-Gruppe in einer bestimmten Anordnung auf.

In den vergangenen Jahren wurden die Anwendungsgebiete für flüssigkristalline Verbindungen auf verschiedene Arten von Anzeigevorrichtungen, elektrooptische Geräte, elektronische Komponenten, Sensoren, etc. erheblich ausgeweitet. Aus diesem Grund wurden eine Reihe verschiedener Strukturen vorgeschlagen, insbesondere auf dem Gebiet der nematischen Flüssigkristalle. Die nematischen Flüssigkristallmischungen haben bisher die breiteste Anwendung in flachen Anzeigevorrichtungen gefunden. Sie wurden besonders in passiven TN- oder STN-Matrixanzeigen oder Systemen mit einer TFT-Aktivmatrix eingesetzt.

Die erfindungsgemäßen Verbindungen können als Komponente(n) flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (electrically controlled birefringence), dem IPS-Effekt (in-plane switching) oder dem Effekt der dynamischen Streuung beruhen.

Die Verwendung bestimmter Derivate mit einer Difluormethylenoxy-Brücke (-CF₂O-) als flüssigkristalline Substanzen ist dem Fachmann bekannt. In der Druckschrift JP 58035174 wird ein Insektizid mit einem Pyridinring und einer CF₂O-Gruppe offenbart.

Darüber hinaus wurden bereits verschiedene Verbindungen mit einer Difluormethylenoxy-Brücke ohne einen Pyridinring als flüssigkristallines Material und dessen Herstellung beschrieben, wie z. B. in der Druckschrift EP 0786445 A1.

Die Druckschrift US 5445763 offenbart smektische Verbindungen mit einem einfach fluorierten Pyridinring zur Verwendung in ferroelektrischen Displays. Das Dokument enthält keine Hinweise auf eine Difluormethylenoxy-Gruppe. Die dort offenbarten Verbindungen sollen die Aufgabe lösen, unpolare Verbindungen oder Verbindungen mit negativer dielektrischer Anisotropie bereitzustellen, nicht jedoch Verbindungen mit einer besonders hohen, positiven dielektrischen Anisotropie.

Die Druckschrift WO 2005/019378 A1 offenbart Flüssigkristallmedien mit dielektrisch positiven Verbindungen, die in der allgemeinen Formel auch Pyridin-Derivate umfassen. Die Beispielverbindungen umfassen auch eine Brückengruppe -CF₂O-. Die Verbindungen werden für einen Anzeigentyp gemäß der vorliegenden Erfindung verwendet. Pyridinverbindungen gemäß der Erfindung werden nicht offenbart.

Der vorliegenden Erfindung lag die Aufgabe zugrunde, neue stabile Verbindungen aufzufinden, die als Komponente(n) flüssigkristalliner Medien geeignet sind. Insbesondere sollen die Verbindungen gleichzeitig eine vergleichsweise geringe Viskosität, sowie eine dielektrische Anisotropie im positiven Bereich besitzen. Für viele aktuelle Mischungskonzepte im Bereich der Flüssigkristalle ist es vorteilhaft, Verbindungen mit einer hohen dielektrischen Anisotropie Δε zu verwenden.

Im Hinblick auf die verschiedensten Einsatzbereiche derartiger Verbindungen mit hohem Δε war es wünschenswert, weitere Verbindungen, vorzugsweise mit hoher Nematogenität zur Verfügung zu haben, die auf die jeweiligen Anwendungen genau maßgeschneiderte Eigenschaften aufweisen.

Der Erfindung lag somit als eine Aufgabe zugrunde, neue stabile, Verbindungen aufzufinden, die als Komponente(n) flüssigkristalliner Medien, insbesondere für z. B. TN-, STN-, IPS- und TN-TFT-Displays, geeignet sind.

Eine weitere Aufgabe der vorliegenden Erfindung war es, Verbindungen bereitzustellen, die für sich oder in Mischungen eine hohe dielektrische Anisotropie Δε sowie einen hohen Klärpunkt aufweisen. Darüber hinaus sollten die erfindungsgemäßen Verbindungen unter den in den Anwendungsgebieten vorherrschenden Bedingungen thermisch und photochemisch stabil sein. Ferner sollten die erfindungsgemäßen Verbindungen möglichst eine breite nematische Phase aufweisen. Als Mesogene sollten sie eine breite nematische Phase in Mischungen mit flüssigkristallinen Cokomponenten ermöglichen sowie hervorragend mit nematischen Basismischungen, insbesondere bei tiefen Temperaturen, mischbar sein.

Überraschenderweise wurde gefunden, dass die erfindungsgemäßen Pyridin-Derivate vorzüglich als Komponenten flüssigkristalliner Medien geeignet sind. Mit ihrer Hilfe lassen sich flüssigkristalline Medien, insbesondere geeignet für TN-TFT- und STN-Displays, aber auch für IPS-Systeme oder neuere Konzepte, die besonders hohe dielektrische Anisotropien benötigen, erhalten. Die erfindungsgemäßen Verbindungen sind besonders stabil, auch unter Lufteinwirkung, und farblos. Auch zeichnen sie sich durch besonders stark positive dielektrische Anisotropien Δε aus, aufgrund derer in der Anwendung in optischen Schaltelementen niedrigere Schwellenspannungen erforderlich sind. Sie besitzen für sich oder in Mischungen einen breiten nematischen Phasenbereich. Darüber hinaus weisen die erfindungsgemäßen Verbindungen einen besonders niedrigen Schmelzpunkt, einen hohen Klärpunkt, sowie gleichzeitig niedere Werte für die Rotationsviskosität γ₁ auf. Im Vergleich mit Stoffen aus dem Stand der Technik wird eine erhöhte thermische Stabilität, ein erhöhter Klärpunkt und eine besonders hohe Polarität (dielektrische Anisotropie) beobachtet.

Mit der Bereitstellung der erfindungsgemäßen Pyridin-Derivate wird ganz allgemein die Palette der flüssigkristallinen Substanzen, die sich unter verschiedenen, anwendungstechnischen Gesichtspunkten zur Herstellung flüssigkristalliner Mischungen eignen, erheblich verbreitert.

Gegenstand der Erfindung sind somit Verbindungen der Formel I, 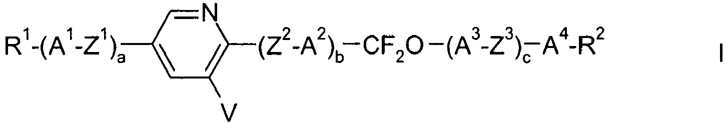 worin
- R¹ und R²: jeweils unabhängig voneinander H, F, Cl, Br, einen halogenierten oder unsubstituierten Alkylrest mit 1 bis 15 C-Atomen, wobei in diesen Resten auch eine oder mehrere CH₂-Gruppen jeweils unabhängig voneinander durch -C≡C-, -CH=CH- , -(CO)O-, -O(CO)-, -(CO)- oder -O-so ersetzt sein können, dass O-Atome nicht direkt miteinander verknüpft sind, wobei R² auch CN, SCN, NCS oder SF₅ bedeuten kann.
- A¹, A², A³ und A⁴: jeweils unabhängig voneinander, gleich oder verschieden:

a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-Phenylen, worin eine oder zwei CH-Gruppen durch N ersetzt sein können und worin auch ein oder mehrere H-Atome gegen Br, Cl, F, CN, Methyl, Methoxy oder eine ein- oder mehrfach fluorierte Methyl- oder Methoxygruppe ersetzt sein können,
   oder
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 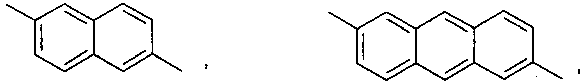 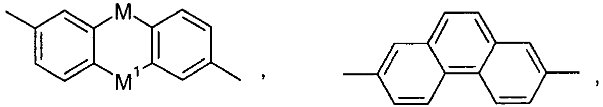 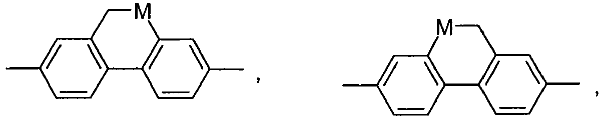 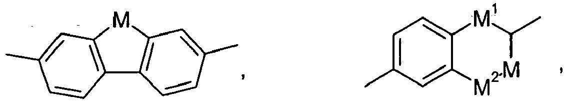 worin Wasserstoffatome ein oder mehrfach durch F, CN, SCN, SF₅, CH₂F, CHF₂, CF₃, OCH₂F, OCHF₂ oder OCF₃ substituiert sein können,
   eine oder mehrere Doppelbindungen durch Einfachbindungen ersetzt sein können,
   M, M¹ oder M² -O-, -S-, -CH₂-, -CHY- oder -CYY¹-, so dass benachbarte Gruppen nicht gleichzeitig -O- oder -S-bedeuten, und
   Y und Y¹ Cl, F, CN, OCF₃ oder CF₃ bedeuten,
   - V: H oder F,
   - Z¹, Z² und Z³: jeweils unabhängig voneinander, gleich oder verschieden, eine Einfachbindung, -CH₂O-, -(CO)O-, -CF₂O-, -CH₂CH₂CF₂O-, -CF₂CF₂-. -CH₂CF₂-, -CH₂CH₂-, -(CH₂)₄-, -CH=CH-, -CH=CF-, -CF=CF- oder -C≡C-, wobei asymmetrische Brücken nach beiden Seiten orientiert sein können, und
   - a: 0, 1 oder 2, bevorzugt 0 oder 1,
   - b: 0, 1 oder 2, bevorzugt 1, und
   - c: 0, 1 oder 2, bevorzugt 0,
   wobei a + b + c ≤ 4, bevorzugt gleich 1, 2 oder 3, besonders bevorzugt 1
   oder 2, ist,
   bedeuten.

A¹⁻³ bzw. Z¹⁻³ können unabhängig auch verschiedene Bedeutungen annehmen wenn sie für a, b oder c > 1 mehrmals auftreten.

Gegenstand der Erfindung ist weiterhin die Verwendung der Verbindungen der Formel I in flüssigkristallinen Medien.

Ebenfalls Gegenstand der vorliegenden Erfindung sind flüssigkristalline Medien mit mindestens zwei flüssigkristallinen Komponenten, welche mindestens ein Pyridin-Derivat der Formel I enthalten.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden für sich oder in Mischungen flüssigkristalline Mesophasen in einem für die elektrooptische Verwendung günstig gelegenen Temperaturbereich. Mit den erfindungsgemäßen Verbindungen können sich breite nematischen Phasenbereiche erzielen lassen. In flüssigkristallinen Mischungen erhöhen die erfindungsgemäßen Verbindungen den Klärpunkt und erhöhen die Polarität der Mischung deutlich. Sie lassen sich auch an Luft während 20 h auf 130 °C oder mehr, bevorzugt sogar auf 150 °C oder mehr erhitzen, ohne dass merkliche Zersetzung eintritt.

Bevorzugt sind Verbindungen der Formel I, worin a 0 oder 1, insbesondere a = 1, ist.
Z¹ und/oder Z³ bedeuten bevorzugt eine Einfachbindung, -CF₂O-, -OCF₂-, -C₂F₄-, -CH₂O-, -OCH₂- oder -(CO)O-, insbesondere eine Einfachbindung. Z² bedeutet bevorzugt -CH₂CH₂-, -CH=CH-, -C=C- oder eine Einfachbindung, insbesondere eine Einfachbindung.

Für den Fall, dass Z² eine Einfachbindung ist, bedeutet A² bevorzugt einen ungesättigten oder aromatischen Ring aus den Gruppen b) oder c) nach der Definition der Formel I.

A¹, A², A³ und A⁴ bedeuten, soweit vorhanden, bevorzugt 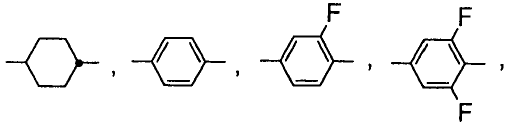 und ferner,

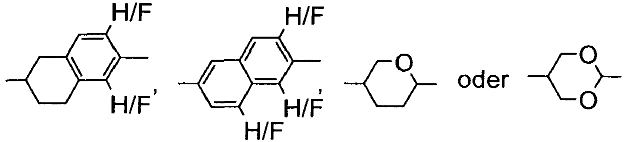

Die Gruppe A¹ bedeutet dabei bevorzugt

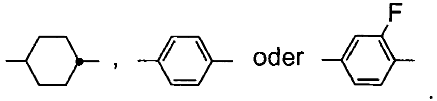

A² bedeutet bevorzugt

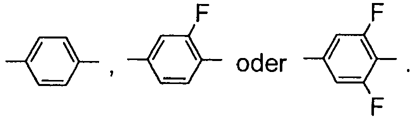

A⁴ bedeutet bevorzugt

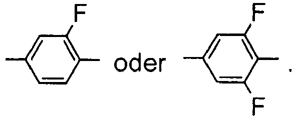

R¹ bedeutet bevorzugt Alkyl, Alkoxy, Alkenyl oder Alkenyloxy mit bis zu 8 Kohlenstoffatomen. R¹ bedeutet besonders bevorzugt geradkettiges Alkyl oder Alkenyl.
Bevorzugt bedeutet R² X, wobei
X F, Cl, OCF₃, OCHF₂, OCHFCF₃, OCF₂CHFCF₃, CF₃, CN, SF₅, NCS,
insbesondere F, Cl, CN oder OCF₃ und ganz besonders F bedeutet. Vorzugsweise bedeuten R¹ und R² nicht gleichzeitig H.

Besonders bevorzugt sind Verbindungen der Formeln IA 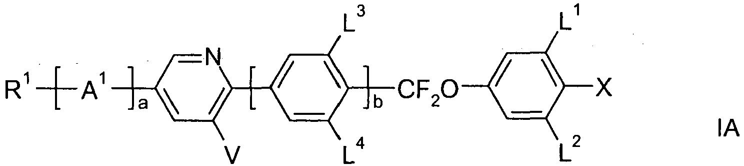 worin
R¹, A¹, X, a, b und V die oben für Formel I angegebenen Bedeutungen haben, sowie
L¹, L², L³ und L⁴
H oder F
bedeuten.

Bevorzugt sind Verbindungen der Formel IA worin L¹ ein Fluor bedeutet. b bedeutet bevorzugt 0 oder 1, insbesondere 1. V ist bevorzugt H. L³ ist bevorzugt F. a + b ist bevorzugt 1, 2 oder 3. Ganz besonders bevorzugt ist b 1, und a ist bevorzugt 1 oder 2. Besonders bevorzugt sind 2, 3 oder vier der Gruppen L¹ bis L⁴ ein Fluor.

In einer weiteren Ausführungsform der Erfindung sind Verbindungen der Formel I bevorzugt, worin V ein F bedeutet und wenigstens einer der Ringe A¹ und A² ein 1,4-Phenylen gemäß der Gruppe b) bedeutet. Besonders bevorzugt sind dabei Verbindungen worin a + b 1, 2 oder 3 beträgt. Insbesondere beträgt b 1 und a 1. Die Gruppe A¹ bedeutet dabei bevorzugt

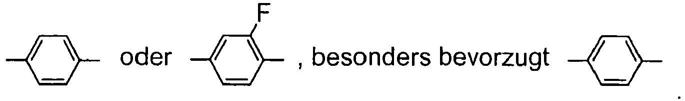

Besonders bevorzugte Verbindungen der Formel I sind die Verbindungen der Formeln I1 bis 17,

| | |
|---|---|
| 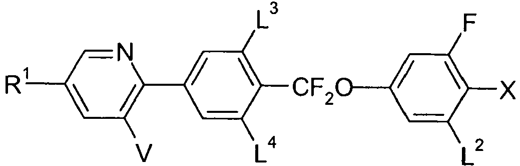 | I1 |
| 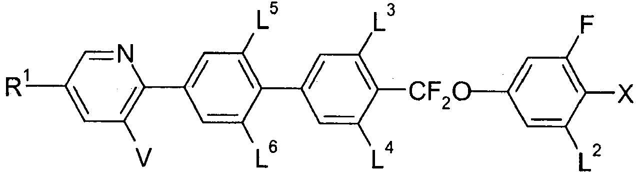 | I2 |
| 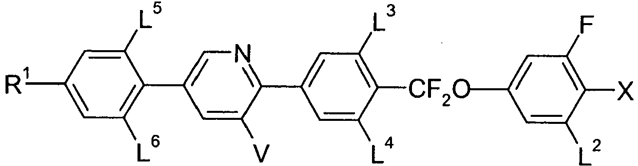 | I3 |
| 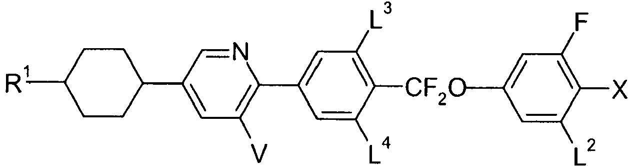 | I4 |
| 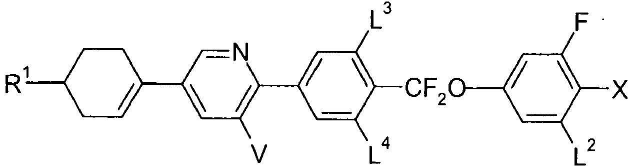 | I5 |
| 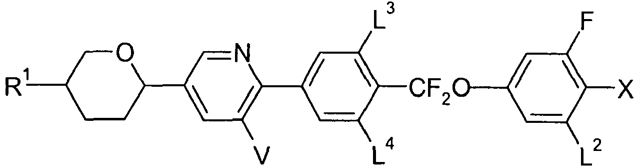 | I6 |
| 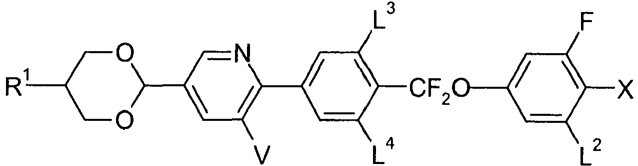 | I7 |

worin R¹, V und X die oben angegebenen Bedeutungen haben. L², L³, L⁴, L⁵ und L⁶ bedeuten unabhängig voneinander H oder F.

Bei Verbindungen, die in Diastereomeren auftreten können, sind sowohl die Reinsubstanzen als auch jedes Mischungsverhältnis der Isomeren umfasst und jeweils als geeignete Mischungskomponente anzusehen.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Verbindungen der Formel I können vorteilhaft wie an den folgenden beispielhaften Synthesen ersichtlich hergestellt werden (Schema 1 und 2):

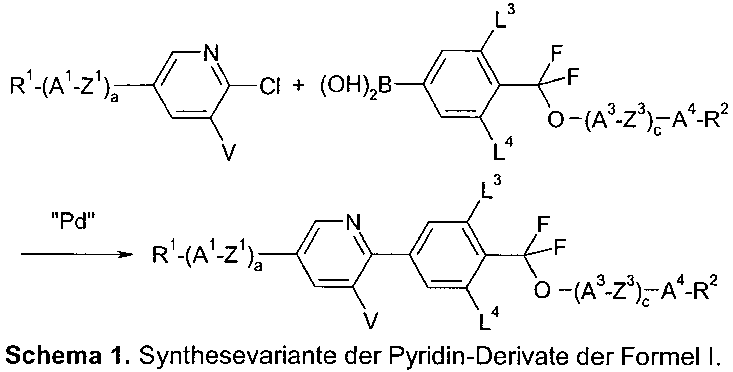

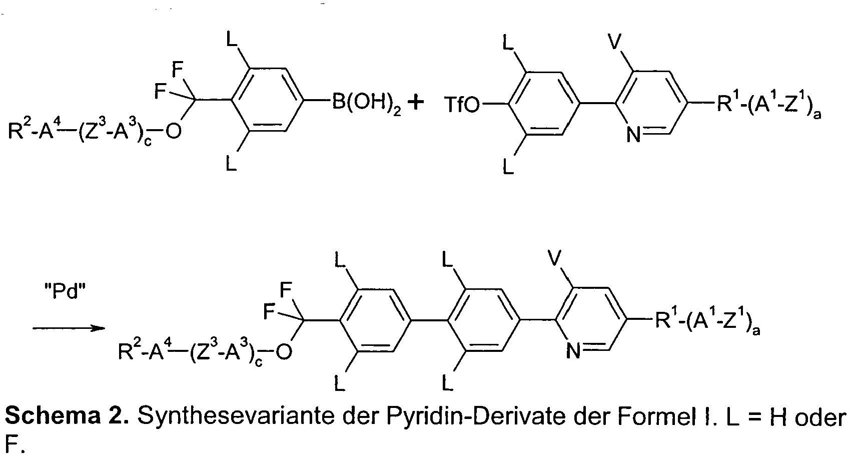

Die unbeteiligten Gruppen der Formeln in Schema 1 und 2 lassen sich variieren, so weit es die Definitionen der Verbindungen der Formel I nahe legen. Entsprechende Ausgangsprodukte lassen sich in der Regel vom Fachmann ohne weiteres herstellen. So lassen sich die Verbindungen der Formeln I oder IA herstellen.

Die Erfindung hat daher auch ein Verfahren zur Herstellung von Verbindungen der Formel I zum Gegenstand:
Ein Verfahren zur Herstellung von Verbindungen der Formel I, worin V Wasserstoff oder Fluor bedeutet, ist dadurch gekennzeichnet, dass es einen Verfahrensschritt umfasst, worin ein 2-substituiertes Pyridin der Formel 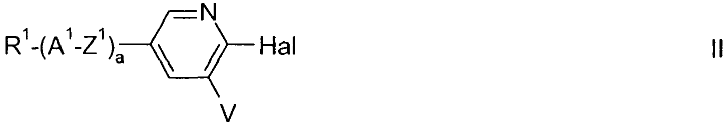 worin R¹, A¹, Z¹, V und a wie in Anspruch 1 definiert sind, und Hal OSO₂CF₃, Cl, Br oder I bedeutet,
   mit einer Boronsäure oder einem offenkettigen oder cyclischen Boronsäureester der Formeln

   (OH)₂B-(Z²-A²)_{b}-CF₂O-(A³-Z³)_{c}-A⁴-R² IIIa

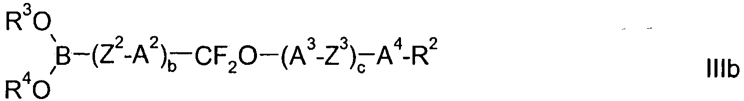

   worin Z², Z³, A², A³, A⁴, b, c und R² wie in Anspruch 1 definiert sind, und
   - R³, R⁴: Alkyl mit 1-12 C-Atomen oder R³+R⁴ zusammen auch ein C₁-C₆-Alkylen, insbesondere der Formeln -CH₂-(CH₂)ₚ-CH₂- und -C(CH₃)₂C(CH₃)₂-,
   oder 1,2-Phenylen bedeuten,
   wobei R³, R⁴ und R³+R⁴ auch substituiert sein können, insbesondere durch C₁-C₆-Alkyl, F, Cl, C₁-C₆-Alkoxy, und wobei p 0 oder 1 ist,
   in Gegenwart eines Übergangsmetallkatalysators, bevorzugt eines Palladiumkomplexes, zur Reaktion gebracht wird. Bei den Komplexen handelt es sich bevorzugt um Palladium(II)-Komplexe, insbesondere um Bis(triphenylphosphin)palladium(II)chlorid. Hal bedeutet bevorzugt Chlor oder Brom, insbesondere Chlor. In IIIa/IIIb bedeutet bevorzugt b 1 oder 2 und Z² eine Einfachbindung. Weiterhin sind die für die Verbindungen der Formel I angegebenen Unterformen bevorzugt.

Weitere bevorzugte Verfahrensvarianten lassen sich den Beispielen entnehmen, deren Details - auch verallgemeinert nach allgemeiner Fachkenntnis - repräsentativ für bevorzugte Ausführungsformen des erfindungsgemäßen Verfahren und seiner Produkte sind.

Gegenstand der Erfindung sind auch flüssigkristalline Medien enthaltend eine oder mehrere der erfindungsgemäßen Verbindungen der Formel I. Die flüssigkristallinen Medien enthalten wenigstens zwei Komponenten. Man erhält sie vorzugsweise indem man die Komponenten miteinander vermischt. Ein Verfahren zur Herstellung eines flüssigkristallinen Mediums ist daher dadurch gekennzeichnet, dass man mindestens eine Verbindung der Formel I mit mindestens einer weiteren mesogenen Verbindung vermischt und gegebenenfalls Additive zugibt.

Die erzielbaren Kombinationen aus Klärpunkt, Viskosität bei tiefer Temperatur, thermischer und UV-Stabilität und dielektrischer Anisotropie übertreffen bei weitem bisherige Materialien aus dem Stand der Technik.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexane, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein. Mischungen für TFT-Displays enthalten vorzugsweise keine Verbindungen aus der Klasse der Carbonester oder der Carbonitrile.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-CF₂O-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Py-, -G-Phe- und -G-Cyc- sowie deren Spiegelbildern gebildeten Gruppe, wobei Phe unsubstituiertes oder durch Fluor substituiertes 1,4-Phenylen, Cyc trans-1,4-Cyclohexylen, Pyr Pyrimidin-2,5-diyl, Dio 1,3-Dioxan-2,5-diyl, Py Tetrahydropyran-2,5-diyl- und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe, Py und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS oder -(O)ᵢCH₃₋ₖFₖ, wobei i 0 oder 1 und k 1, 2 oder 3 ist.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋ₖ Fₖ, wobei i 0 oder 1 und k 1, 2 oder 3 ist. Die Verbindungen, in denen R" diese Bedeutung hat, werden mit den Teilformeln 1 b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1 b, 2b, 3b, 4b und 5b hat R' die bei den Verbindungen der Teilformeln 1a bis 5a angegebenen Bedeutungen und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a bis 5a angegebenen Bedeutungen und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen in den erfindungsgemäßen Medien sind vorzugsweise:

| | |
|---|---|
| Gruppe A: | 0 bis 90 %, vorzugsweise 20 bis 90 %, besonders bevorzugt 30 bis 90 %; |
| Gruppe B: | 0 bis 80 %, vorzugsweise 10 bis 80 %, besonders bevorzugt 10 bis 65 %; |
| Gruppe C: | 0 bis 80 %, vorzugsweise 0 bis 80 %, besonders bevorzugt 0 bis 50 %; |

wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 %, der erfindungsgemäßen Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z.B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe, chirale Dotierstoffe, Stabilisatoren oder Nanopartikel zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben. Die erfindungsgemäßen Flüssigkristallmischungen ermöglichen eine bedeutende Erweiterung des zur Verfügung stehenden Parameterraumes.

Gegenstand der Erfindung sind auch elektrooptische Anzeigen (insbesondere TFT-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Der Ausdruck "Alkyl" umfasst geradkettige und verzweigte Alkylgruppen mit 1-9 Kohlenstoffatomen, insbesondere die geradkettigen Gruppen Methyl, Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" umfasst geradkettige und verzweigte Alkenylgruppen mit bis zu 9 Kohlenstoffatomen, insbesondere die geradkettigen Gruppen. Besonders bevorzugte Alkenylgruppen sind C₂₋C₇-1E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1 E-Propenyl, 1E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "halogenierter Alkylrest" umfasst vorzugsweise ein- oder mehrfach fluorierte und/oder chlorierte Reste. Perhalogenierte Reste sind eingeschlossen. Besonders bevorzugt sind fluorierte Alkylreste, insbesondere CF₃, CH₂CF₃, CH₂CHF₂, CHF₂, CH₂F, CHFCF₃ und CF₂CHFCF₃.

Der Ausdruck "Alkylen" umfasst geradkettige und verzweigte Alkandiylgruppen mit 1-12 Kohlenstoffatomen, insbesondere die geradkettigen Gruppen Methylen, Ethylen, Propylen, Butylen und Pentylen. Gruppen mit 2-8 Kohlenstoffatomen sind im Allgemeinen bevorzugt.

Die Gesamtmenge an Verbindungen der Formeln I in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formel I ist.

Der Aufbau der erfindungsgemäßen Matrix-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der Matrix-Anzeige, insbesondere auch Matrix-Anzeigeelemente auf Basis von poly-Si TFT.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die folgenden Beispiele erläutern die Erfindung, ohne sie begrenzen zu sollen. Vor- und nachstehend bedeuten Prozentangaben Gewichtsprozent. Alle Temperaturen sind in Grad Celsius angegeben. Ferner bedeuten Tg Glastemperatur, K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm, 20 °C), Δε die dielektrische Anisotropie (1 kHz, 20 °C) und γ₁ die Rotationsviskosität (in der Einheit mPa·s).

Die die Substituenten an den gezeichneten gesättigten 1,4-substituierten Ringsystemen der Synthesebeispiele sind, soweit nicht anders angegeben, trans-konfiguriert. Die übrigen Formeln stehen für beide Konfigurationen und bevorzugt für die trans-Konfiguration

Die Bestimmung physikalischer, physikochemischer beziehungsweise elektrooptischer Parameter erfolgt nach allgemein bekannten Verfahren, wie sie unter anderem beschrieben sind in der Broschüre "Merck Liquid Crystals - Licristal® - Physical Properties of Liquid Crystals - Description of the Measurements Methods", 1998, Merck KGaA, Darmstadt.
Die dielektrische Anisotropie Δε der einzelnen Substanzen wird bei 20 °C und 1 kHz bestimmt. Dazu werden 5-10 Gew.% der zu untersuchenden Substanz in der dielektrisch positiven Mischung ZLI-4792 (Merck KGaA) gelöst gemessen und der Messwert auf eine Konzentration von 100 % extrapoliert. Die optische Anisotropie Δn wird bei 20°C und einer Wellenlänge von 589,3 nm bestimmt, die Rotationsviskosität γ₁ bei 20°C, beide ebenfalls durch lineare Extrapolation. Der Klärpunkt wird an der Reinsubstanz oder, wenn das nicht möglich ist, ebenfalls durch Extrapolation aus ZLI-4792 bestimmt.

Folgende Abkürzungen werden verwendet:

| | |
|---|---|
| p-TsOH | *p*-Toluolsulfonsäure |
| THF | Tetrahydrofuran |
| MTB-Ether | Methyl-*t*-butylether |
| RT | Raumtemperatur |
| BuLi | *n*-Buthyllithium |
| DMAP | *N*,*N*-Dimethylaminopyridin |
| DCC | Dicyclohexylcarbodiimid |
| OBN | Benzyloxy-Substituent |
| DC | Dünnschichtchromatographie |
| DAST | Diethylaminoschwefeltrifluorid |
| Pd/C | Palladium-Katalysator auf Träger (Kohle, ca. 5 % Pd) |

### Beispiel 1

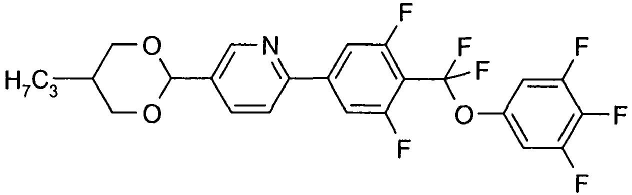

### Schritt 1.1

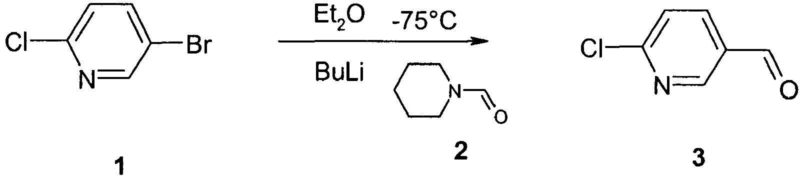

Eine Lösung des Pyridins **1** (25,0 g; 120 mmol) in 300 ml Diethylether wird unter Stickstoff bei -70 °C mit 85 ml 15%igem BiLi in *n*-Hexan versetzt. Nach 90 min wird ebenfalls bei tiefer Temperatur eine Lösung von 13,7 ml (120 mmol) Formylpiperidin (**2**) in den Ansatz gegeben. Nach einer weiteren Stunde wird der Ansatz auf -10 °C erwärmt, mit Wasser versetzt und mit MTB-Ether verdünnt. Die organische Phase wird über Natriumsulfat getrocknet und eingeengt. Der erhaltene Rückstand wird über Kieselgel geben (MTB-Ether/*n*-Heptan 1:1). Anschließend erfolgte eine Kristallisation aus *n*-Heptan bei -20 °C.

### Schritt 1.2

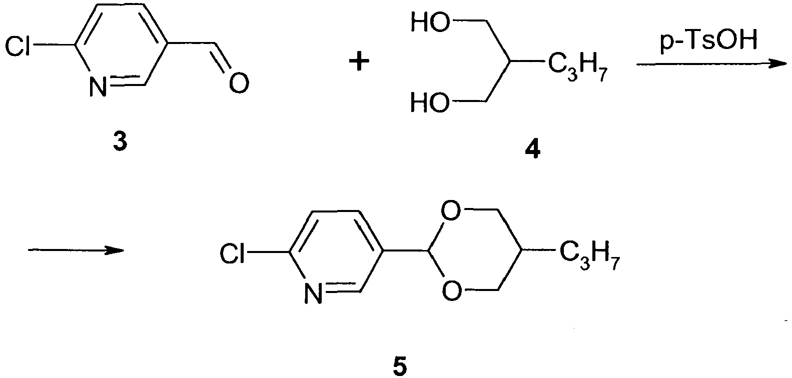

13,1 g (111 mmol) des Diols **4**, 17,5 g (90%ig; 111 mmol) des Aldehyds **3** und 1 g p-Toluolsulfonsäure werden mit 100 ml Toluol versetzt und 3 h am Wasserabscheider erhitzt. Der abgekühlte Ansatz wird über Kieselgel (Toluol) gegeben. Das erhaltene Produkt wird ohne weiter Reinigung in der Folgestufe eingesetzt.

### Schritt 1.3

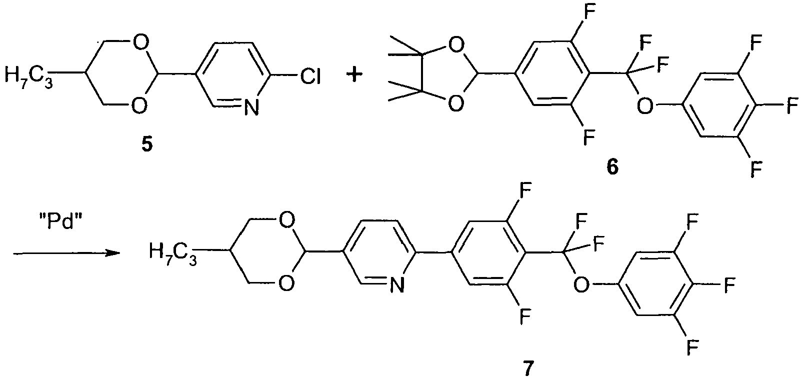

11,1 g (40 mmol) Natriummetaborat-Octahydrat werden in 32 ml Wasser vorgelegt und mit 40 ml THF, 0,1 ml (0,7 mmol) Hydraziniumhydroxid und 0,6 g (0,8 mmol) Bis(triphenylphosphin)-palladium(II)chlorid versetzt und 5 min bei RT gerührt. Anschließend wird eine Lösung von 17,4 g (40 mmol) des Boronsäureesters **6** und 9,7 g (40 mmol) des Chlorids **5** in den Ansatz gegeben. Nach 16 h unter Rückfluss wird das Reaktionsgemisch mit MTB-Ether verdünnt. Die organische Phase wird eingeengt. Der Rückstand wird über Kieselgel (Toluol) filtriert. Die Endreinigung des Produktes erfolgt durch Kristallisation aus EtOH/MTB-Ether.
K 125 SmA (107) N 137 I
Δε 39
Δn 0,141
Analog wird hergestellt: 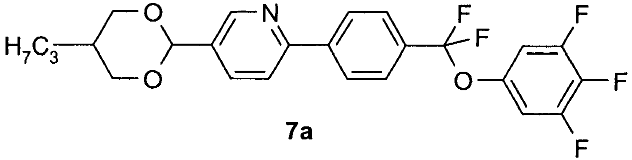 K 138 SmA 207 N 209 I 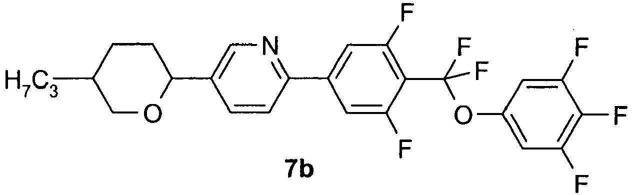 K 57 SmA 58 N 126 I
Δε 33
Δn 0,160

### Beispiel 2

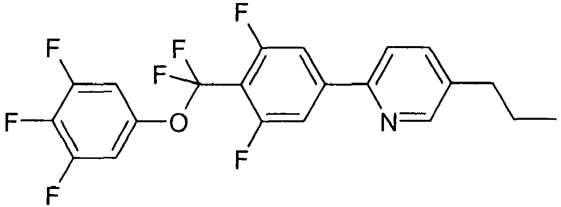

### Schritt 2.1

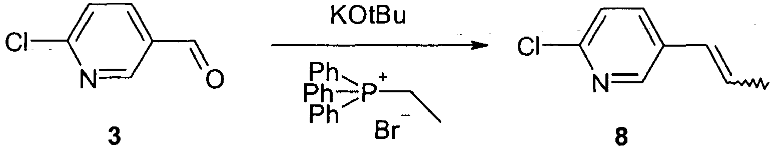

33,6 g (90 mmol) des Wittig-Salzes und 14,8 g (86%ig; 90 mmol) des Aldehyds **3** werden in 140 ml THF suspendiert und bei einer Temperatur unter 20 °C portionsweise mit 10,1 g (90 mmol) Kalium-*tert*-butylat versetzt. Der Ansatz wird über Nacht bei RT gerührt. Nach der Zugabe von Wasser wird der Ansatz mit n-Heptan extrahiert. Die organische Phase wird eingeengt und mit n-Heptan/MTB-Ether (7:3) über Kieselgel filtriert. Man erhält eine gelbe Flüssigkeit **8**.

### Schritt 2.2

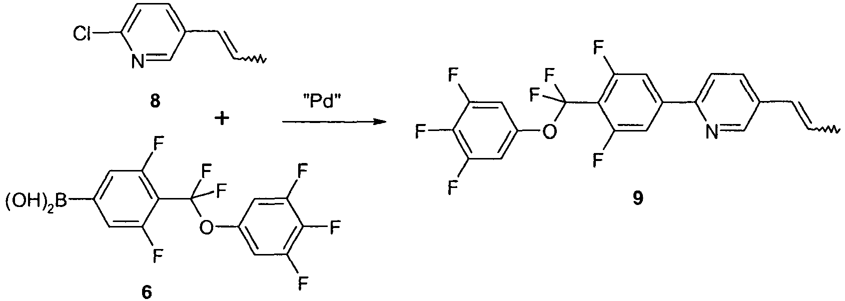

11,1 g (40 mmol) Natriummetaborat-Octahydrat werden in 32 ml Wasser vorgelegt und mit 32 ml THF, 0,1 ml (0,7 mmol) Hydraziniumhydroxid und 0,6 g (0,8 mmol) Bis(triphenylphosphin)-palladium(II)chlorid versetzt und 5 min bei RT gerührt. Anschließend wird eine Lösung von 40,9 g (35%ig; 40 mmol) der Boronsäure **6** und 6,7 g (43,8 mmol) des Chlorids **8** in den Ansatz gegeben. Nach 16 h unter Rückfluss wird das Reaktionsgemisch mit MTB-Ether verdünnt. Die organische Phase wird eingeengt. Der Rückstand wird über Kieselgel (n-Heptan) filtriert. Die Endreinigung des Produktes erfolgt durch Kristallisation aus Heptan.

### Schritt 2.3

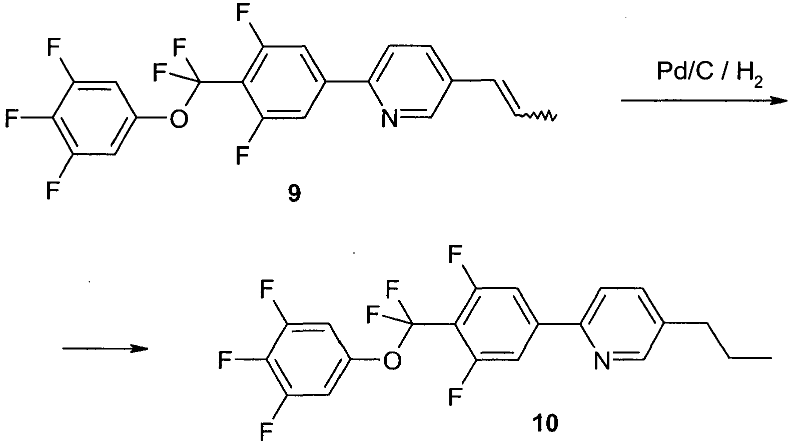

7,0 g (90%ig, 12 mmol) des Alkens **9** werden in THF gelöst und am Palladiumkatalysator (5 % Pd auf Kohle) hydriert. Anschließend wird die Lösung eingeengt, und der Rückstand über Kieselgel (Toluol/n-Heptan 1:1) gegeben. Die weitere Aufreinigung erfolgt durch Kristallisation aus n-Heptan.
K 37 l
Δε 31
Δn 0,127
γ₁ 65 mPa·s

Analog wird hergestellt: 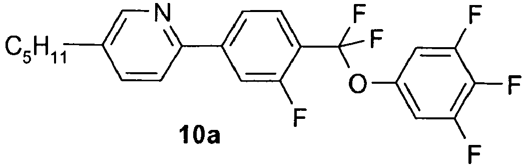 K 38 SmA (6) N (13) I
Δε 25
Δn 0,141
γ₁ 100 mPa·s 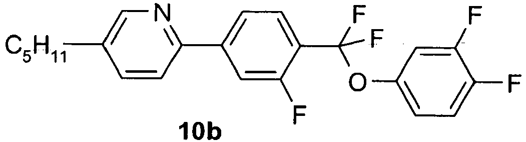 Tg -71 K 36 SmA (-5) N (33) I
Δε 20
Δn 0,157
γ₁ 136 mPa·s 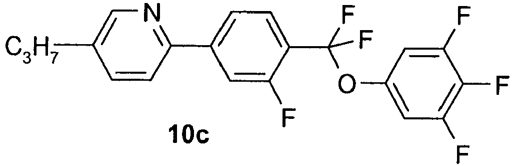 K 40 SmA (-4) N (0) I
Δε 27
Δn 0,144
γ₁ 97 mPa·s

### Beispiel 3

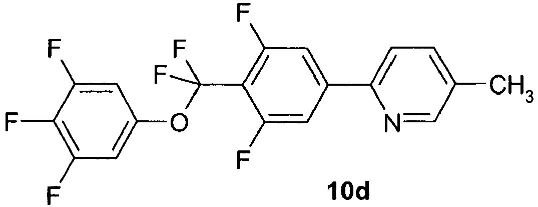

Analog zu Beispiel 2, Schritt 2.2 wird die Verbindung **10d** aus der Boronsäure **6** und 2-Chlor-5-Methylpyridin hergestellt.
K 78 l
Δε 35
Δn 0,137
γ₁ 69 mPa·s

Wiederum analog wird die folgende Verbindung **10e** hergestellt: 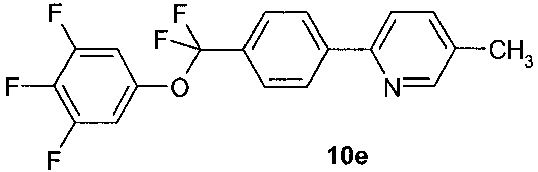 K 122 I
Δε 27
Δn 0,182
γ₁ 76 mPa·s

### Beispiel 4

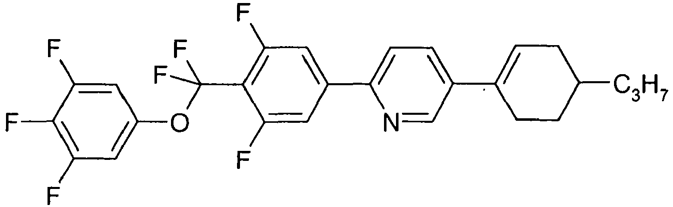

### Schritt 4.1

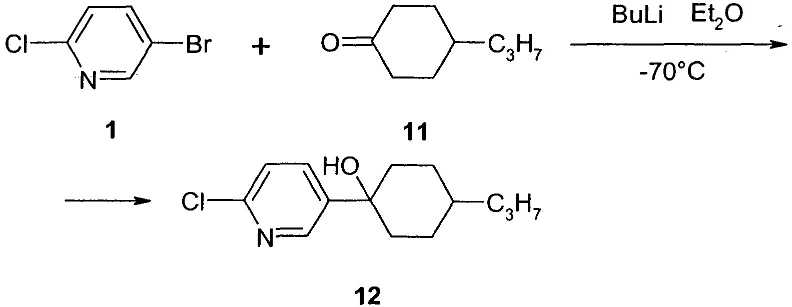

Eine Lösung des Pyridins **1** (50,0 g ; 260 mmol) in 400 ml Diethylether wird unter Stickstoff bei -70 °C mit 180 ml (290 mmol) 15%igem BuLi in *n-*Hexan versetzt. Nach 60 min wird ebenfalls bei tiefer Temperatur eine Lösung von 36,4 g (260 mmol) des Ketons **11** in 200 ml Diethylether in den Ansatz gegeben. Nach einer weiteren Stunde wird der Ansatz auf -20 °C erwärmt, auf Eiswasser gegeben. Die organische Phase wird über Natriumsulfat getrocknet und eingeengt. Der erhaltene Rückstand wird ohne weitere Reinigung in der Folgestufe eingesetzt.

### Schritt 4.2

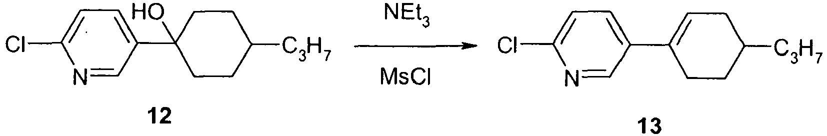

Unter Stickstoff werden 66 g (260,0 mmol) des Alkohols **12** in 800 ml Dichlormethan und 108 ml Triethylamin gelöst und bei 0 °C mit 26,2 ml (340 mmol) Methansulfonsäurechlorid (MsCl) versetzt. Der Ansatz wird über Nacht bei RT gerührt. Anschließend wird das Reaktionsgemisch auf Wasser gegeben und mit MTB-Ether extrahiert. Die organische Phase wird eingeengt, und der erhaltene Rückstand über Kieselgel gegeben (MTB-Ether/n-Heptan 1:4). Der Rückstand wird ohne weitere Reinigung in der folgenden Stufe eingesetzt.

### Schritt 4.3

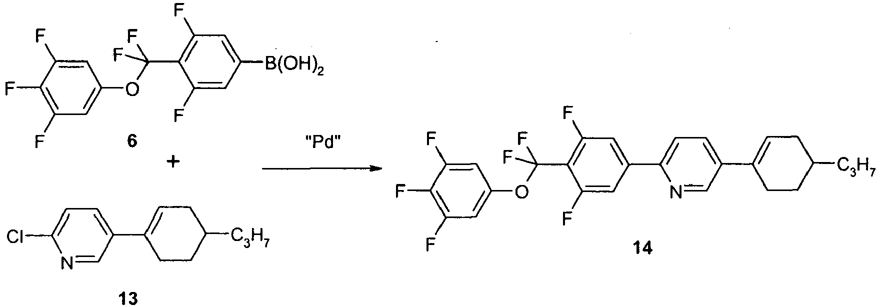

8,7 g (30 mmol) Natriummetaborat-Octahydrat werden in 15 ml Wasser vorgelegt und mit 40 ml THF, 0,10 ml Hydraziniumhydroxid und 300 mg Bis(triphenylphosphin)-palladium(II)chlorid versetzt und 5 min bei RT gerührt. Anschließend wird eine Lösung von 21,5 g (35%ig; 20 mmol) der Boronsäure **6** und 4,7 g (20 mmol) des Chlorids **13** in den Ansatz gegeben. Nach 15 h unter Rückfluss wird das Reaktionsgemisch mit MTB-Ether extrahiert. Die organische Phase wird eingeengt. Der Rückstand wird über Kieselgel (n-Heptan) filtriert. Die Endreinigung des Produktes erfolgt durch Kristallisation aus Heptan.
K 73 SmA (73) N 138 I
Δε 30
Δn 0,197

Analog wird hergestellt: 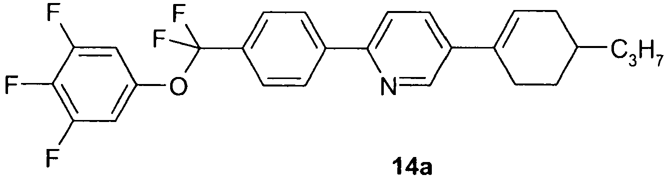 K 100 SmC 107 SmA 184 N 195 I
Δε 24
Δn 0,215 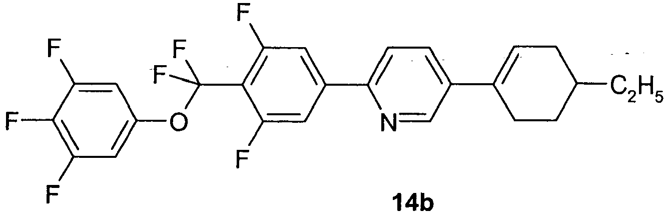 K 74 SmA (50) N 111 I
Δε 32
Δn 0,191 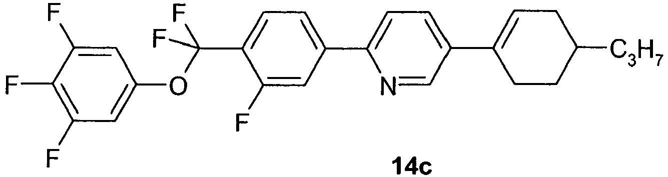 K 54 SmC 55 SmA 136 N 168 I
Δε 26
Δn 0,211 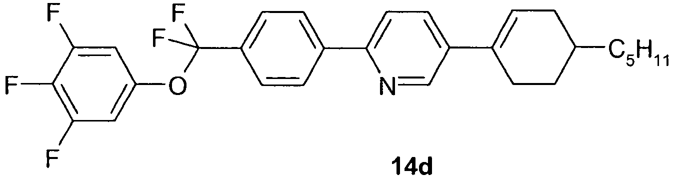 K 87 SmC 103 SmA 179 N 192 I
Δε 23
Δn 0,213 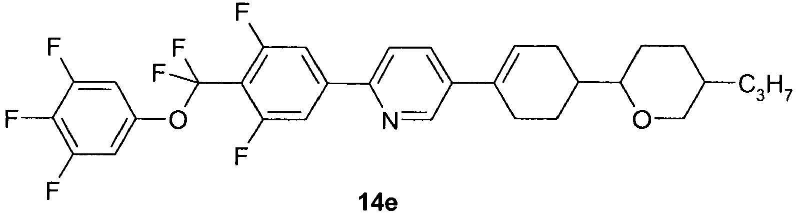 K 81 SmC 125 N 257 I
Δε 33
Δn 0,209 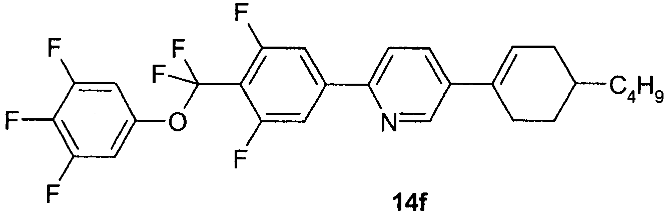 K 62 SmA 82 N 133 I
Δε 29
An 0,194
γ₁ 402 mPa·s

### Beispiel 5

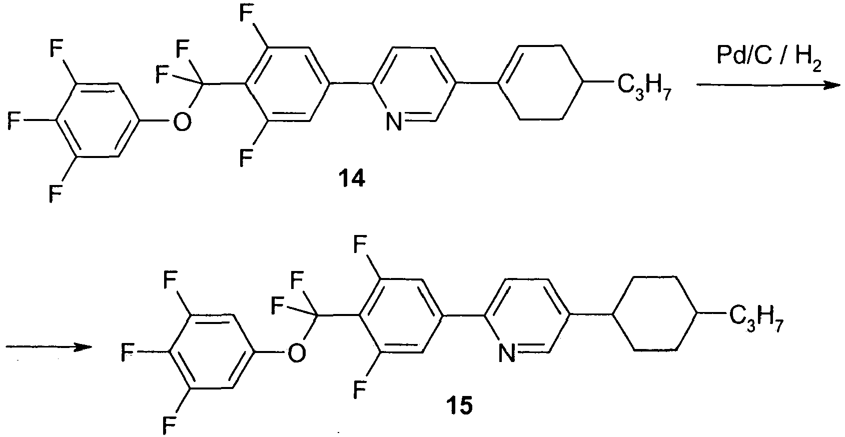

7,0 g (90%ig, 12 mmol) des Alkens **14** (vgl. Beispiel 4) werden in THF gelöst und am Palladiumkatalysator hydriert. Anschließend wird die Lösung eingeengt, und der Rückstand über Kieselgel (Toluol/n-Heptan 1:1) gegeben. Die weitere Aufreinigung erfolgt durch Kristallisation aus n-Heptan.
K 73 N 137 I
Δε 28
Δn 0,156

Analog wird hergestellt: 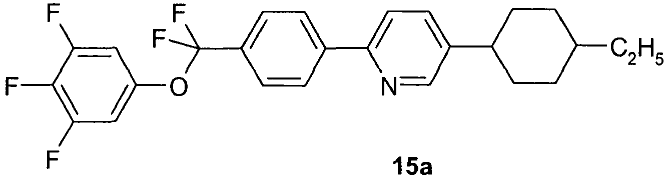 K 99 SmA 177 N 174 I
Δε 22
Δn 0,170 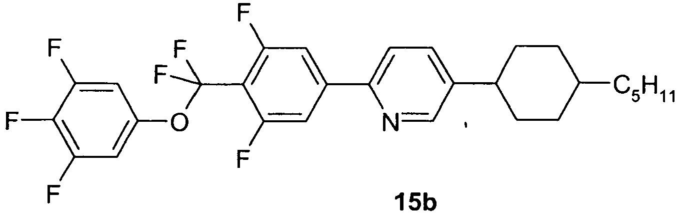 K 51 N 137 I
Δε 28
Δn 0,159 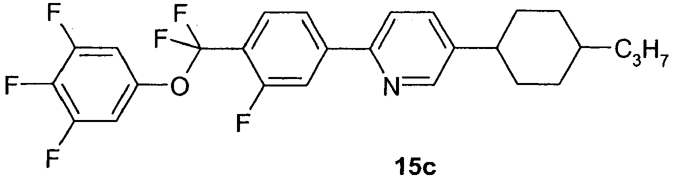 K 73 N 172 I
Δε 24
Δn 0,171 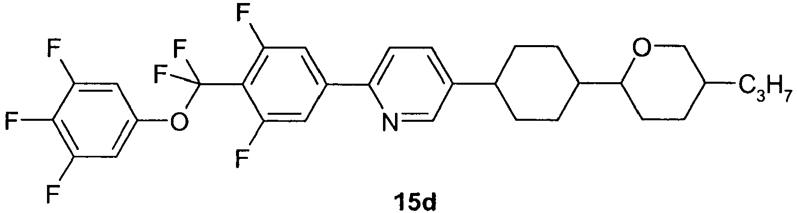 K 93 N 260 I
Δε 31
Δn 0,169 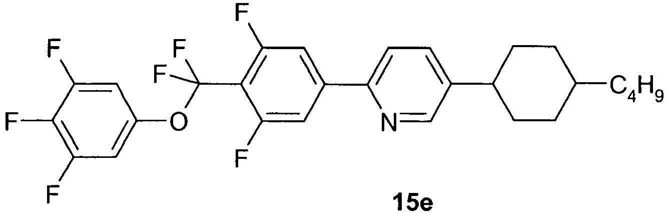 K 52 N 133 I
Δε 27
Δn 0,149

### Beispiel 6

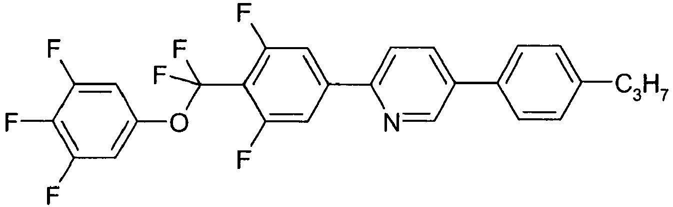

### Schritt 6.1

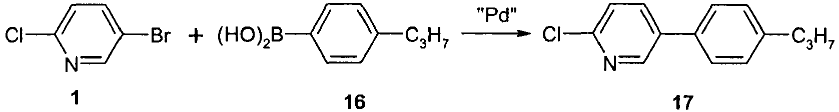

Eine Mischung von 100 ml Toluol und 50 ml 2 N Natriumcarbonatlösung wird mit 0,7 g Tetrakis(triphenylphosphin)-palladium, 8,2 g (50 mmol) der Boronsäure **16** und 9,6 g (50 mmol) des Pyridins **1** versetzt. Nach 60 h bei 60 °C wird das Reaktionsgemisch mit MTB-Ether verdünnt. Die organische Phase wird eingeengt. Der Rückstand wird über Kieselgel (n-Heptan) filtriert.

### Schritt 6.2

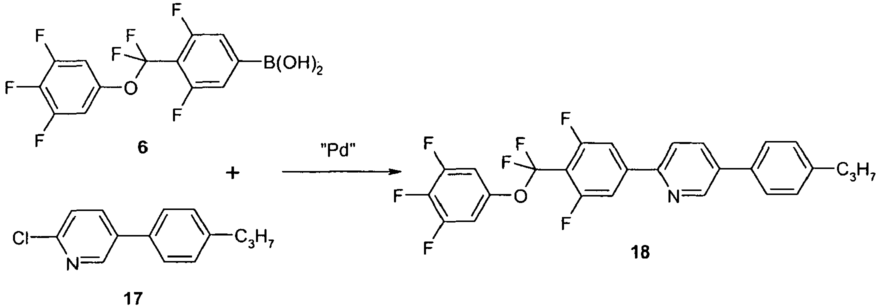

12,6 g (45 mmol) Natriummetaborat-Octahydrat werden in 23 ml Wasser vorgelegt und mit 25 ml THF, 0,1 ml (0,7 mmol) Hydraziniumhydroxid und 0,7 g (1 mmol) Bis(triphenylphosphin)-palladium(II)chlorid versetzt und 5 min bei RT gerührt. Anschließend wird eine Lösung von 10,5 g (30 mmol) der Boronsäure **6** und 7,0 g (30 mmol) des Chlorids **17** in den Ansatz gegeben. Nach 16 h unter Rückfluss wird das Reaktionsgemisch mit MTB-Ether verdünnt. Die organische Phase wird eingeengt. Der Rückstand wird über Kieselgel(n-Heptan) filtriert. Die Endreinigung des Produktes erfolgt durch Kristallisation aus Heptan.
K 81 SmA 106 N 143 I
Δε 33
Δn 0,236

Analog wird hergestellt: 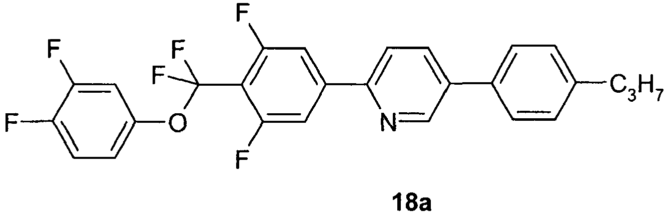 K 83 SmA 128 N 168 I
Δε 26
Δn 0,250 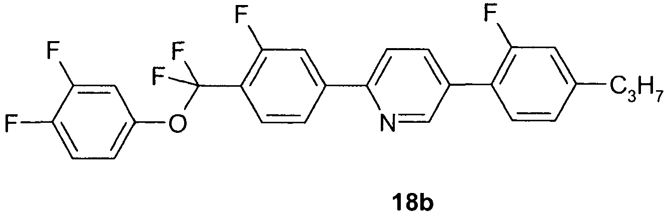 K 50 SmA 130 N 177 I
Δε 25
Δn 0,241 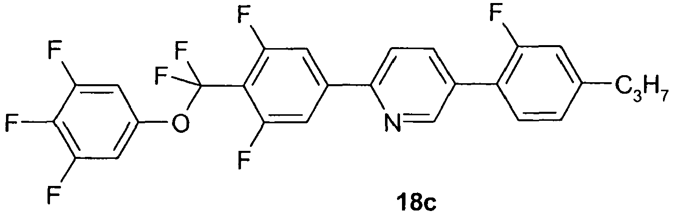 K 85 SmA (82) N 123 I
Δε 36
Δn 0,213

### Beispiel 7

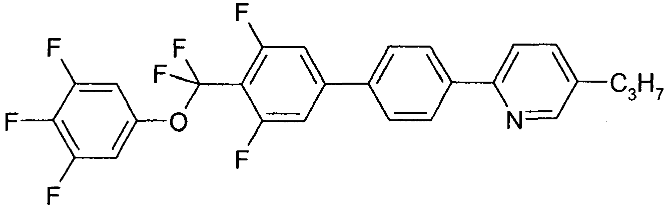

### Schritt 7.1

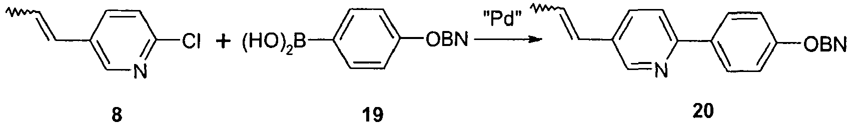

21 g (75 mmol) Natriummetaborat-Octahydrat werden in 38 ml Wasser vorgelegt und mit 40 ml THF, 0,15 ml (1 mmol) Hydraziniumhydroxid und 0,7 g (1 mmol) Bis(triphenylphosphin)-palladium(II)chlorid versetzt und 5 min bei RT gerührt. Anschließend wird eine Lösung von 11,4 g (50 mmol) der 4-Benzyloxyphenylboronsäure **19** und 7,7 g (50 mmol) des Chlorids **8** in den Ansatz gegeben. Nach 6 h unter Rückfluss wird das Reaktionsgemisch mit MTB-Ether verdünnt. Die organische Phase wird eingeengt. Der Rückstand wird über Kieselgel (n-Heptan) filtriert.

### Schritt 7.2

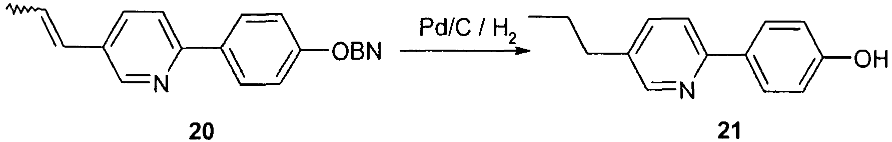

7,5 g (25 mmol) des Alkens **20** werden in THF gelöst und am Palladiumkatalysator hydriert. Anschließend wird die Lösung eingeengt, und der Rücktand über Kieselgel (Toluol/MTB-Ether 1:1) gegeben.

### Schritt 7.3

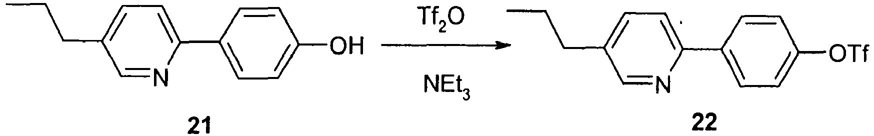

7,5 g (25 mmol) des Phenols **21** werden in 700 ml Dichlormethan gelöst und mit 55 ml Triethylamin und 700 mg Dimethylaminopyridin versetzt. Anschließend werden bei 5 °C 41 ml (25 mmol) Trifluormethansulfonsäureanhydrid (Tf₂O) innerhalb von 30 min tropfenweise zugegeben. Nach 17 h bei RT wird der Ansatz mit n-Heptan verdünnt und über Kieselgel gegeben (MTB-Ether Heptan 1:2). Das Produkt wird ohne weitere Reinigung in der Folgestufe eingesetzt.

### Schritt 7.4

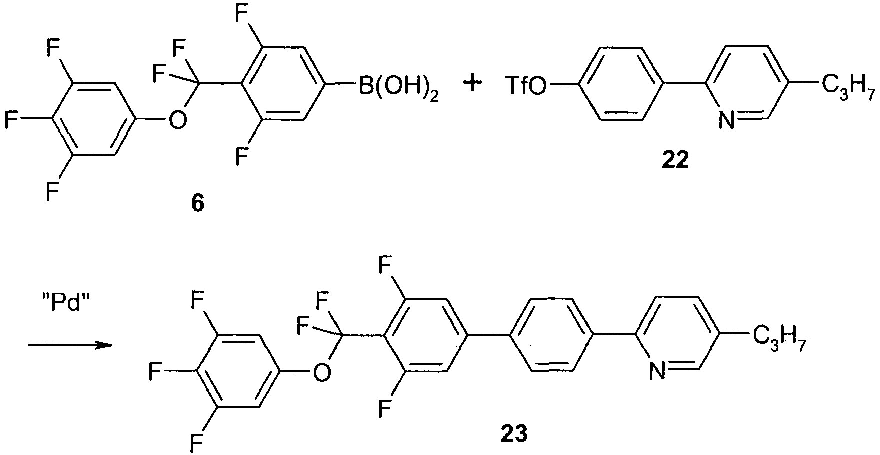

8,4 g (30 mmol) Natriummetaborat-Octahydrat werden in 15 ml Wasser vorgelegt und mit 15 ml THF, 0,15 ml (1 mmol) Hydraziniumhydroxid und 0,7 g (1 mmol) Bis(triphenylphosphin)-palladium(II)chlorid versetzt und 5 min bei RT gerührt. Anschließend wird eine Lösung von 7,0 g (20 mmol) der Boronsäure **6** und 6,9 g (20 mmol) des Triflats **22** in den Ansatz gegeben. Nach 16 h unter Rückfluss wird das Reaktionsgemisch mit MTB-Ether verdünnt. Die organische Phase wird eingeengt. Der Rückstand wird über Kieselgel (n-Heptan/Toluol) filtriert. Die Endreinigung des Produktes **23** erfolgt durch Kristallisation aus Heptan.

### Beispiel 8

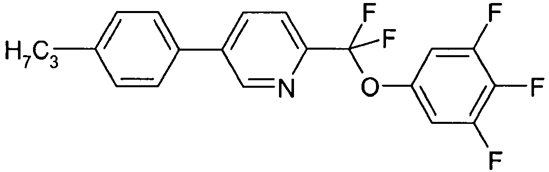

### Schritt 8.1

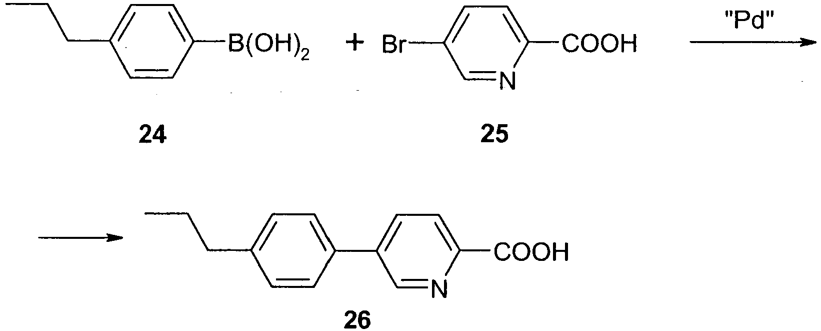

8,4 g (30 mmol) Natriummetaborat-Octahydrat werden in 15 ml Wasser vorgelegt und mit 15 ml THF, 0,15 ml (1 mmol) Hydraziniumhydroxid und 0,7 g (1 mmol) Bis(triphenylphosphin)-palladium(II)chlorid versetzt und 5 min bei RT gerührt. Anschließend wird eine Lösung von 3,3 g (20 mmol) der Boronsäure **24** und 4,1 g (20 mmol) des Bromids **25** in den Ansatz gegeben. Nach 16 h unter Rückfluss wird das Reaktionsgemisch mit MTB-Ether verdünnt und auf pH 6 eingestellt. Die organische Phase wird eingeengt. Der Rückstand wird ohne weitere Reinigung in der Folgestufe eingesetzt.

### Schritt 8.2

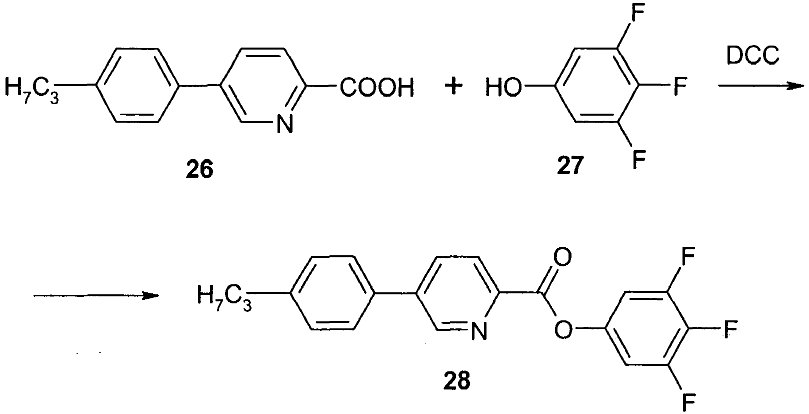

12,1 g (50 mmol) der Säure **26**, 7,4 g (50 mmol) des Phenols **27** und 270 mg DMAP werden unter Stickstoff in 70 ml Toluolvorgelegt, auf 0 °C abgekühlt und bei max. 5 °C mit einer Lösung von 11,3 g (55 mmol) DCC in 300 ml Toluol versetzt. Der Ansatz wird über Nacht bei RT gerührt. Anschließend werden 6,1 g Oxalsäure in das Gemisch gegeben und nochmals 1 h bei RT gerührt. Das Reaktionsgemisch wird auf 0 bis 5 °C abgekühlt, und der ausgefallene Feststoff abgetrennt. Das Filtrat wird am Rotationsverdampfer zum Rückstand eingeengt. Der Rückstand wird über Kieselgel gegeben (MTB/Heptan 1:1).

### Schritt 8.3

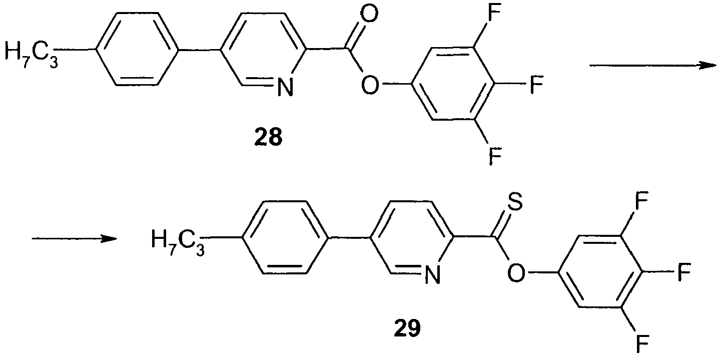

37,1 g (100 mmol) des Esters **28** und 50,6 g (130 mmol) Lawesson's Reagenz (2,4-Bis(4-methoxyphenyl)-1,3,2,4-dithiadiphosphetan-2,4-disulfid) werden mit 500 ml Chlorbenzol versetzt und bis zum vollständigen Umsatz des Esters **28** (DC-Kontrolle) zum Sieden erhitzt. Der abgekühlte Ansatz wird über Kiesel abgesaugt und das Filtrat eingeengt. Der Rückstand wird an Kieselgel gereinigt.

### Schritt 8.4

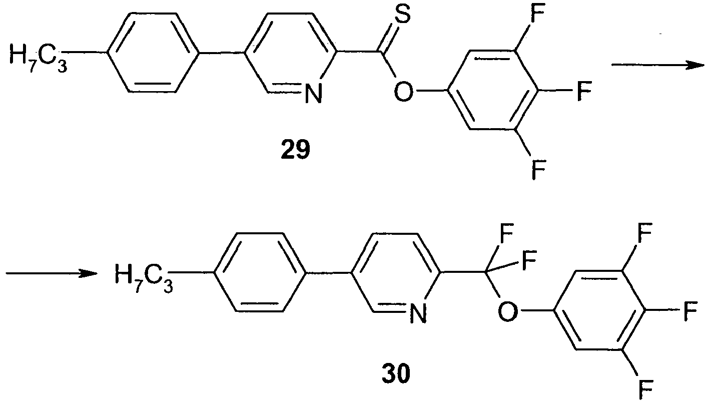

7,7 g (20 mmol) des Thioesters **29** werden in 40 ml Dichlormethan gelöst und bei 20° mit 26,7 ml (20 mmol) DAST versetzt und 48 h bei 60° gerührt. Der abgekühlte Ansatz wird auf gesättigte NaHCO₃-Lösung gegossen und mit Dichlormethan extrahiert. Die organische Phase wird über Natriumsulfat getrocknet und einrotiert. Der Rückstand wird an Kieselgel gereinigt (MTB-Ether/Heptan 1:2).

### Beispiele 9 a-f)

Analog zu den Beispielen 1, 3, 4, 5, 6 und 7 werden unter Verwendung des Ausgangsmaterials der Formel (**31**) 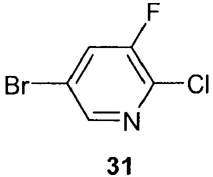 anstelle des Pyridins **1** die folgenden Verbindungen erhalten:
9.a) Analog Beispiel 1: 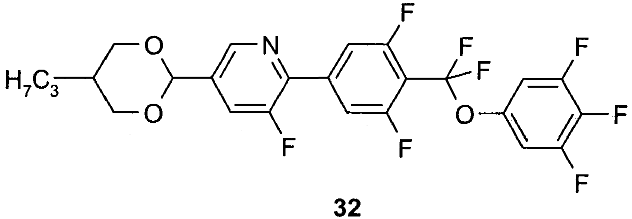
9.b) Analog Beispiel 3:

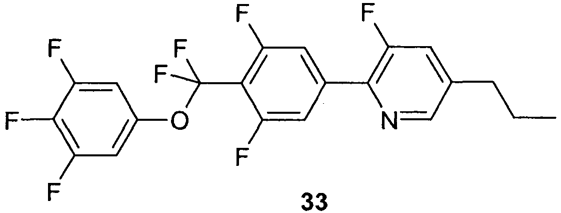

### Schritt 9b.1

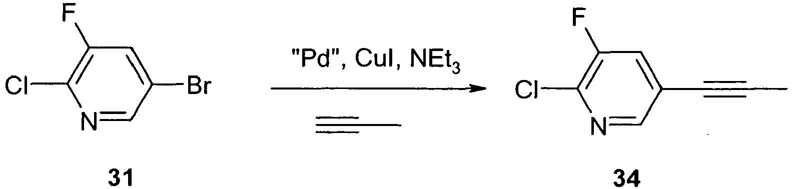

21,1 g (100 mmol) 5-Brom-2-chlor-3-fluorpyridin **31** werden in 300 ml THF und 340 ml Triethylamin gelöst. Dazu werden bei 0 °C 6,1 g (150 mmol) Propin eingeleitet. Anschließend wird mit 2,8 g (4 mmol) Bis(triphenylphosphin)-palladium(II)chlorid und 380 mg (2,0 mmol) Cu(I)iodid versetzt und 12 h bei RT gerührt. Das Rohprodukt **34** wird eingeengt und mit n-Heptan/MTB-Ether (8:2) über Kieselgel filtriert. Man erhält ein dunkles Öl, das im nächsten Schritt eingesetzt wird.

### Schritt 9b.2

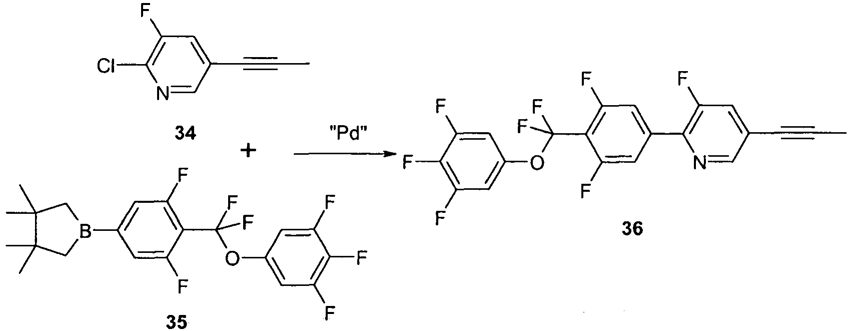

8,7 g (30 mmol) Natriummetaborat-Octahydrat werden in 24 ml Wasser vorgelegt und mit 32 ml THF, 0,1 ml (0,6 mmol) Hydraziniumhydroxid und 0,44 g (0,60 mmol) Bis(triphenylphosphin)-palladium(II)chlorid versetzt und 5 min bei RT gerührt. Anschließend wird eine Lösung von 13,1 g (30 mmol) des Boronsäureesters **35** und 7,27 g (30 mmol) des Chlorpyridins **34** gelöst in 32 ml THF in den Ansatz gegeben. Nach 8 h unter Rückfluss wird das Reaktionsgemisch mit MTB-Ether verdünnt. Die organische Phase wird eingeengt. Der Rückstand wird über 100 ml Kieselgel (n-Heptan/MTB-Ether) filtriert. Die Endreinigung der Verbindung **36** erfolgt durch Kristallisation aus Heptan.
K 90 N (72) I
Δε 42
Δn 0,211

### Schritt 9b.3

Das Alkin **36** wird analog Schritt 2.3 an der Endgruppe zum gewünschten Produkt **33** hydriert.
K 32 I
Δε 37
Δn 0,126

### 9.c) Analog Beispiel 4:

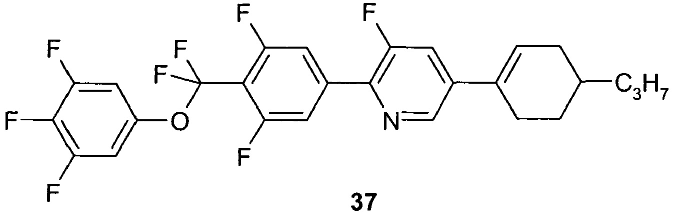

### Schritt 9c.1

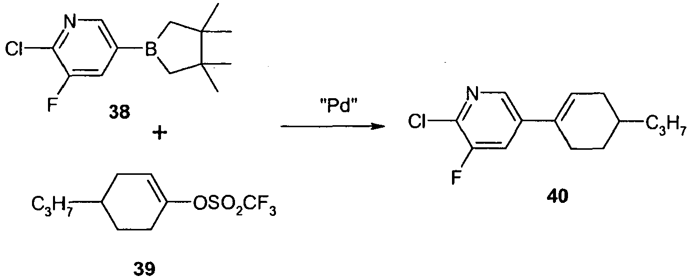

Analog zu Schritt 9b.2 werden 16,6 g (63,1 mmol) des Boronsäureesters **38** (aus **31** durch Pd-Kupplung mit Bis-pinacolato-dibor) mit 21,8 g (80 mmol) des Cyclohexen-Triflats **39** unter Palladiumkatalyse zur Verbindung **40** umgesetzt und aufgearbeitet.

### Schritt 9c.2

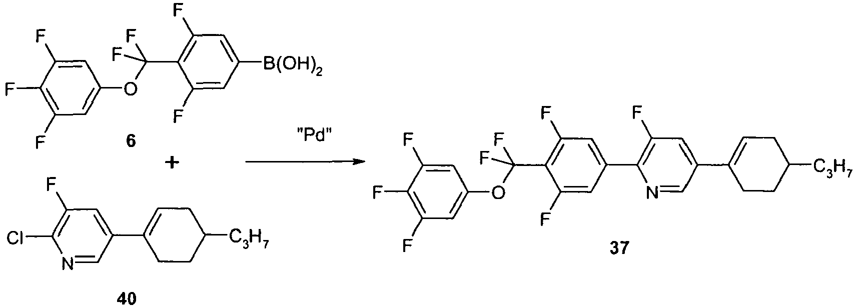

Die Umsetzung von **40** mit **6** zum gewünschten Produkt **37** erfolgt analog Schritt 4.3.

### 9.d) Analog Beispiel 5:

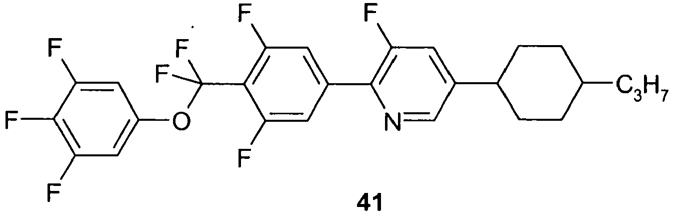

Die Verbindung **37** wird analog Beispiel 5 zur Verbindung **41** hydriert.

### 9.e) Analog Beispiel 6:

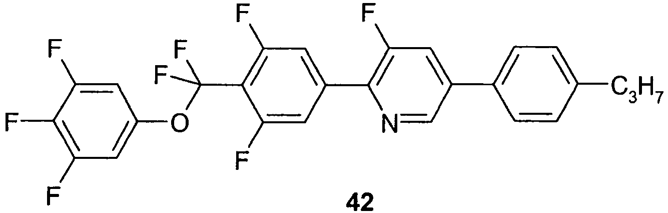

Tg -51 K 60 SmA 85 N 128 I
Δε 38
Δn 0,226
γ₁ 250 mPa·s 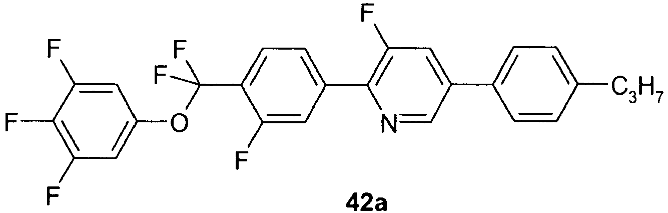 K 70 SmA 117 N 155 I
Δε 33
Δn 0,240

### 9.f) Analog Beispiel 7:

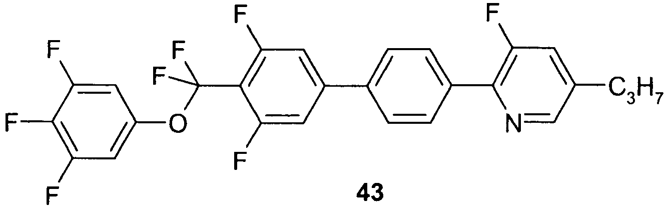

Weitere Kombinationen der Ausführungsformen und Varianten der Erfindung gemäß der Beschreibung ergeben sich auch aus den folgenden Ansprüchen.

## Patentansprüche

1. Verbindungen der Formel I, 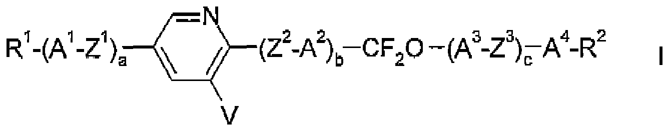 worin
R¹ und R² jeweils unabhängig voneinander H, F, Cl, Br, einen halogenierte oder unsubstituierten Alkylrest mit 1 bis 15 C-Atomen, wobei in diesen Resten auch eine oder mehrere CH₂-Gruppen jeweils unabhängig voneinander durch -C≡C-, -CH=CH-, -(CO)O-, -O(CO)-, -(CO)- oder -O- so ersetzt sein können, dass O-Atome nicht direkt miteinander verknüpft sind, wobei R² auch CN, SCN, NCS oder SF₅ bedeuten kann.
A¹, A², A³ und A⁴ jeweils unabhängig voneinander, gleich oder verschieden:
a) trans-1,4-Cyclohexylen oder Cyclohexenylen, worin auch eine oder mehrere nicht benachbarte CH₂-Gruppen durch -O- und/oder -S- ersetzt sein können und worin H durch F substituiert sein kann,
b) 1,4-phenylen, worin eine oder zwei CH-Gruppen durch N ersetzt sein können und worin auch ein oder mehrere H-Atome gegen Br, Cl, F, CN, Methyl, Methoxy oder eine ein- oder mehrfach fluorierte Methyl- oder Methoxygruppe ersetzt sein können,
oder
c) ein Rest aus der Gruppe 1,4-Bicyclo(2,2,2)-octylen, Piperidin-1,4-diyl, Cylcobut-1,3-diyl, Spiro[3.3]heptan-2,6-diyl, 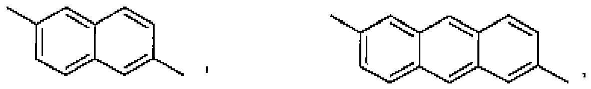 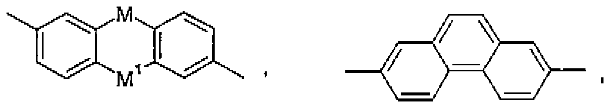 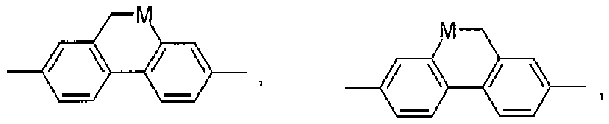 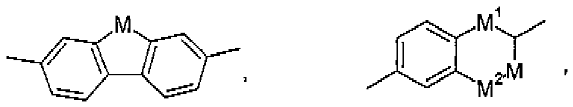 worin Wasserstoffatome ein oder mehrfach durch F, CN, SCN, SF₅, CH₂F, CHF₂, CF₃, OCH₂F, OCHF₂ oder OCF₃ substituiert sein können,
eine oder mehrere Doppelbindungen durch Einfachbindungen ersetzt sein können, M, M¹ oder M² -O-, -S-, -CH₂-, -CHY- oder -CYY¹-, so dass benachbarte Gruppen nicht gleichzeitig -O- oder -S-bedeuten, und
Y und Y¹ Cl, F, CN, OCF₃ oder CF₃ bedeuten,
V H oder F,
Z¹ , Z² und Z³ jeweils unabhängig voneinander, gleich oder verschieden,
eine Einfachbindung, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- oder -C≡C-, wobei asymmetrische Brücken nach beiden Seiten orientiert sein können, und
a 0, 1 oder 2,
b 0, 1 oder 2, und
c 0, 1 oder 2
wobei a + b + c ≤ 4 ist,
bedeuten.

2. Verbindungen nach Anspruch 1 der Formel IA 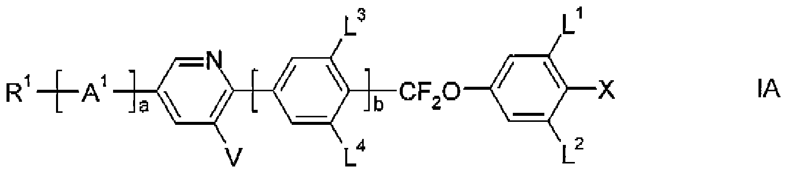 worin
R¹, A¹, a, b und V die in Anspruch 1 für Formel I angegebenen Bedeutungen haben,
X F, OCF₃, CN, CF₃, SCN, SF, NCS, Cl, OCHF₂, OCHFCF₃, OCF₂CHFCF₃,
V H oder F, und
L¹, L², L³ und L⁴ jeweils unabhängig voneinander H oder F,
bedeuten.

3. Verbindungen nach Anspruch 1 oder 2, **dadurch gekennzeichnet, dass**
R¹ Alkyl, Alkoxy, Alkenyl oder Alkenyloxy mit bis zu 8 Kohlenstoffatomen bedeutet.

4. Verbindungen nach einem oder mehreren der Ansprüche 2 bis 3, **dadurch gekennzeichnet, dass** L¹ Fluor und L² unabhängig Fluor oder Wasserstoff bedeuten.

5. Verbindungen nach einem oder mehreren der Ansprüche 1 bis 4 der Formeln I1 bis I7,
| | |
|---|---|
| 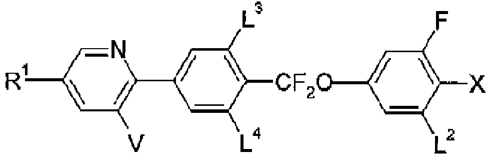 | I1 |
| 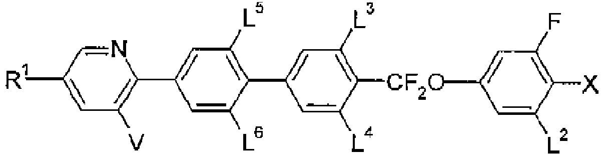 | I2 |
| 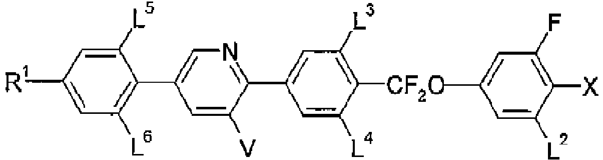 | I3 |
| 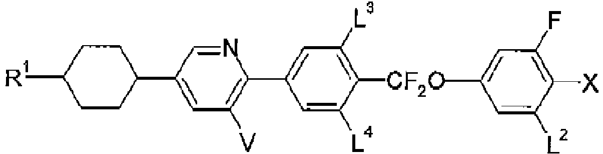 | I4 |
| 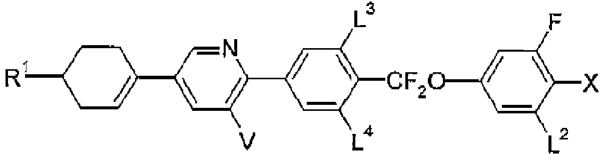 | I5 |
| 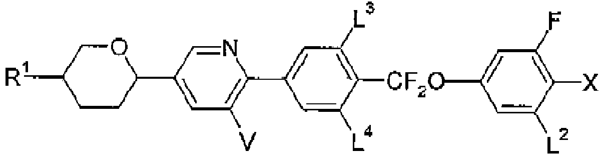 | I6 |
| 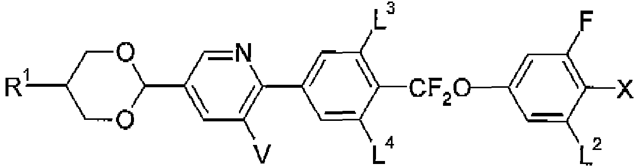 | I7 |
worin R¹ und V die in Anspruch 1 angegebenen Bedeutungen haben und
X F, OCF₃, CN, CF₃, SCN, SF₅, NCS, Cl, OCHF₂, OCHFCF₃, OCF₂CHFCF₃ und
L², L³, L⁴, L⁵ und L⁶
H oder F
bedeuten.

6. Verbindungen nach einem oder mehreren der Ansprüche 2 bis 5, **dadurch gekennzeichnet, dass** L¹ und L² Fluor bedeuten.

7. Verbindungen nach einem oder mehreren der Ansprüche 1 bis 6, **dadurch gekennzeichnet, dass** V Wasserstoff bedeutet.

8. Verbindungen nach einem oder mehreren der Ansprüche 1 bis 6, **dadurch gekennzeichnet, dass** V Fluor bedeutet.

9. Verfahren zur Herstellung von Verbindungen der Formel I nach einem oder mehreren der Ansprüche 1 bis 8, worin V Fluor oder Wasserstoff bedeutet, **dadurch gekennzeichnet, dass** es einen Verfahrensschritt umfasst, worin ein 2-substituiertes Pyridin der Formel 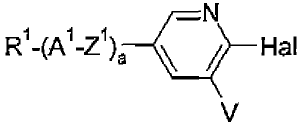 worin R¹, A¹, Z¹ und a wie in Anspruch 1 definiert sind, und Hal OSO₂CF₃, Cl, Br oder I bedeutet,
mit einer Boronsäure oder einem offenkettigen oder cyclischen Boronsäuresäureester der Formeln
(OH)₂B-(Z²-A²)_{b}-CF₂O-(A³-Z³)_{c}-A⁴-R² 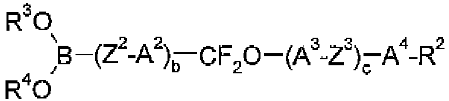
worin Z², Z³, A², A³, A⁴, a, b und R² wie in Anspruch 1 definiert sind, und
R³, R⁴ Alkyl mit 1-12 C-Atomen oder R³+R⁴ zusammen auch ein C₂-C₈-Alkylen oder ein 1,2-Phenylen bedeuten,
wobei R³, R⁴ und R³+R⁴ auch substituiert sein können,
in Gegenwart eines Übergangsmetallkatalysators zur Reaktion gebracht wird.

10. Verwendung einer oder mehrerer Verbindungen der Formel I nach einem oder mehreren der Ansprüche 1 bis 8 als Komponenten in einem flüssigkristallinen Medium.

11. Flüssigkristallines Medium enthaltend mindestens zwei mesogene Verbindungen, **dadurch gekennzeichnet, dass** es mindestens eine Verbindung der Formel I nach einem oder mehreren der Ansprüche 1 bis 8 enthält.

12. Verwendung des flüssigkristallinen Mediums nach Anspruch 11 für elektrooptische Zwecke.

13. Elektrooptische Flüssigkristallanzeige enthaltend ein flüssigkristallines Medium nach Anspruch 11.

## Claims

1. Compounds of the formula I, 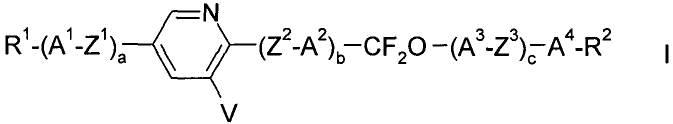 in which
R¹ and R² each, independently of one another, denote H, F, Cl, Br, a halogenated or unsubstituted alkyl radical having 1 to 15 C atoms, where, in addition, one or more CH₂ groups in these radicals may each be replaced, independently of one another, by -C≡C-, -CH=CH-, -(CO)O-, -O(CO)-, -(CO)- or -O- in such a way that O atoms are not linked directly to one another, where R² may also denote CN, SCN, NCS or SF₅,
A¹, A², A³ and A⁴ each, independently of one another, identically or differently, denote:
a) trans-1,4-cyclohexylene or cyclohexenylene, in which, in addition, one or more non-adjacent CH₂ groups may be replaced by -O- and/or -S- and in which H may be substituted by F,
b) 1,4-phenylene, in which one or two CH groups may be replaced by N and in which, in addition, one or more H atoms may be replaced by Br, Cl, F, CN, methyl, methoxy or a mono- or polyfluorinated methyl or methoxy group,
or
c) a radical from the group 1,4-bicyclo[2.2.2]octylene, piperidine-1,4-diyl, cyclobutane-1,3-diyl, spiro[3.3]-heptane-2,6-diyl, 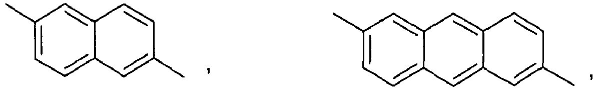 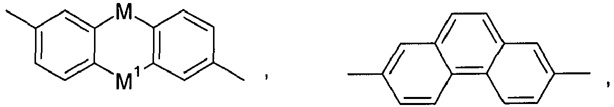 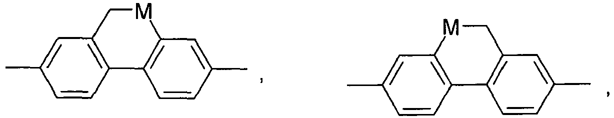 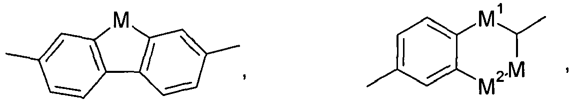 in which hydrogen atoms may be mono- or polysubstituted by F, CN, SCN, SF₅, CH₂F, CHF₂, CF₃, OCH₂F, OCHF₂ or OCF₃,
one or more double bonds may be replaced by single bonds,
M, M¹ or M² denotes -O-, -S-, -CH₂-, -CHY- or -CYY¹- in such a way that adjacent groups do not simultaneously denote -O- or -S-, and
Y and Y¹ denote Cl, F, CN, OCF₃ or CF₃,
V denotes H or F,
Z¹, Z² and Z³ each, independently of one another, identically or differently, denote a single bond, -CH₂O- -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- or -C≡C-, where asymmetrical bridges may be oriented to both sides, and
a denotes 0, 1 or 2,
b denotes 0, 1 or 2, and
c denotes 0, 1 or 2,
where a + b + c is ≤ 4.

2. Compounds according to Claim 1 of the formula IA 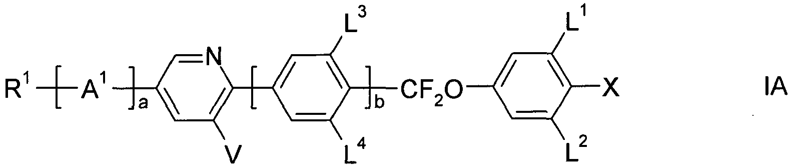 in which
R¹, A¹, a, b and V have the meanings indicated for formula I in Claim 1,
X denotes F, OCF₃, CN, CF₃, SCN, SF₅, NCS, Cl, OCHF₂, OCHFCF₃, OCF₂CHFCF₃,
V denotes H or F, and
L¹, L², L³ and L⁴ each, independently of one another, denote H or F.

3. Compounds according to Claim 1 or 2, **characterised in that**
R¹ denotes alkyl, alkoxy, alkenyl or alkenyloxy having up to 8 carbon atoms.

4. Compounds according to one or more of Claims 2 to 3, **characterised in that** L¹ denotes fluorine and L² independently denotes fluorine or hydrogen.

5. Compounds according to one or more of Claims 1 to 4 of the formulae 11 to 17,
| | |
|---|---|
| 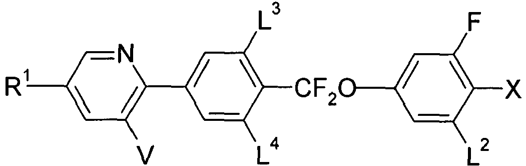 | I1 |
| 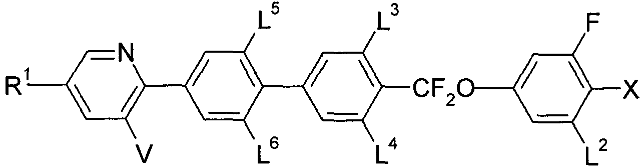 | I2 |
| 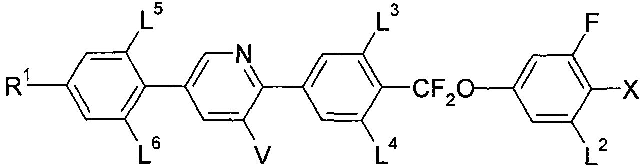 | I3 |
| 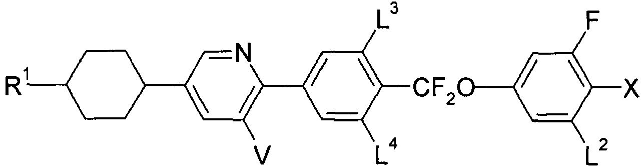 | I4 |
| 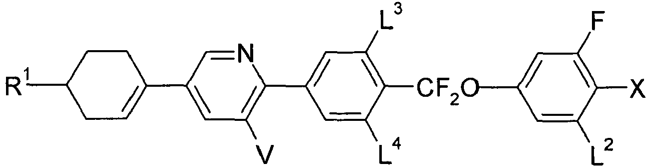 | I5 |
| 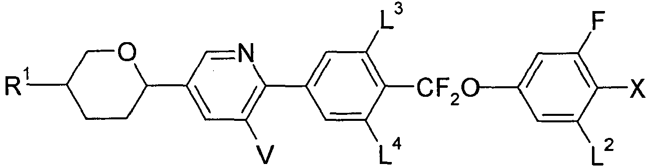 | I6 |
| 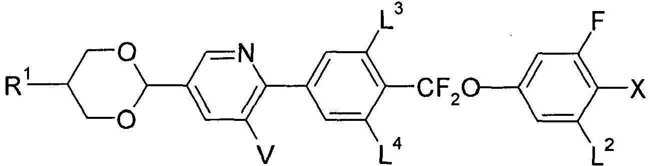 | I7 |
in which R¹ and V have the meanings indicated in Claim 1, and
X denotes F, OCF₃, CN, CF₃, SCN, SF₅, NCS, Cl, OCHF₂, OCHFCF₃, OCF₂CHFCF₃ and
L², L³, L⁴, L⁵ and L⁶
denote H or F.

6. Compounds according to one or more of Claims 2 to 5, **characterised in that** L¹ and L² denote fluorine.

7. Compounds according to one or more of Claims 1 to 6, **characterised in that** V denotes hydrogen.

8. Compounds according to one or more of Claims 1 to 6, **characterised in that** V denotes fluorine.

9. Process for the preparation of compounds of the formula I according to one or more of Claims 1 to 8 in which V denotes fluorine or hydrogen, **characterised in that** it comprises a process step wherein a 2-substituted pyridine of the formula 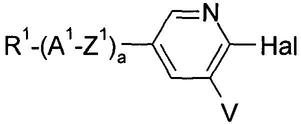 in which R¹, A¹, Z¹ and a are as defined in Claim 1, and
Hal denotes OSO₂CF₃, Cl, Br or I,
is brought to reaction with a boronic acid or an open-chain or cyclic boronic acid ester of the formulae
(OH)₂B-(Z²-A²)_{b}-CF₂O-(A³-Z³)_{c}-A⁴-R² 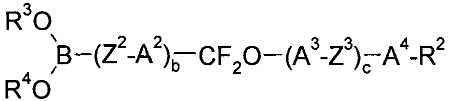
in which Z², Z³, A², A³, A⁴, a, b and R² are as defined in Claim 1, and
R³, R⁴ denote alkyl having 1-12 C atoms or R³+R⁴ together also denote a C₂-C₈-alkylene or a 1,2-phenylene,
where R³, R⁴ and R³+R⁴ may also be substituted,
in the presence of a transition-metal catalyst.

10. Use of one or more compounds of the formula I according to one or more of Claims 1 to 8 as components in a liquid-crystalline medium.

11. Liquid-crystalline medium comprising at least two mesogenic compounds, **characterised in that** it comprises at least one compound of the formula I according to one or more of Claims 1 to 8.

12. Use of the liquid-crystalline medium according to Claim 11 for electro-optical purposes.

13. Electro-optical liquid-crystal display containing a liquid-crystalline medium according to Claim 11.

## Revendications

1. Composés de la formule I : 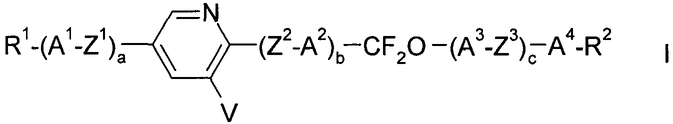 dans laquelle :
R¹ et R² représentent, chacun indépendamment de l'autre, H, F, Cl, Br, un radical alkyle halogéné ou non substitué comportant de 1 à 15 atomes de C, où, en outre, un ou plusieurs groupe(s) CH₂ dans ces radicaux peuvent chacun être remplacés, indépendamment les uns des autres, par -C≡C-, -CH=CH-, -(CO)O-, -O(CO)-, -(CO)- ou -O- de telle sorte que des atomes de O ne soient pas liés directement les uns aux autres, où R² peut également représenter CN, SCN, NCS ou SF₅,
A¹, A², A³ et A⁴ représentent, chacun indépendamment des autres, de manière identique ou différente :
a) trans-1,4-cyclohexylène ou cyclohexénylène, où, en outre, un ou plusieurs groupes CH₂ non adjacents peuvent être remplacés par -O- et/ou -S- et où H peut être substitué par F,
b) 1,4-phénylène, où un ou deux groupe(s) CH peut/ peuvent être remplacé(s) par N et où, en outre, un ou plusieurs atome(s) de H peut/peuvent être remplacé(s) par Br, C!, F, CN, méthyle, méthoxy ou un groupe méthyle ou méthoxy monofluoré ou polyfluoré,
ou
c) un radical pris parmi le groupe 1,4-bicyclo[2.2.2]-octylène, pipéridine-1,4-diyle, cyclobutane-1,3-diyle, spiro[3.3]heptane-2,6-diyle, 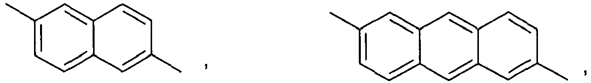 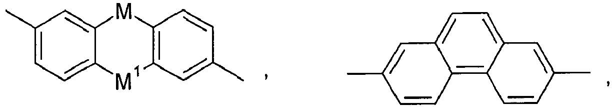 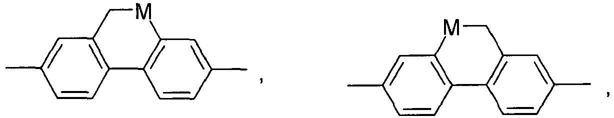 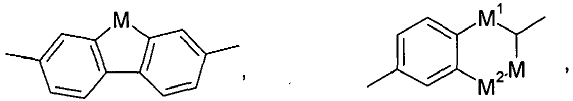 où des atomes d'hydrogène peuvent être monosubstitués ou polysubstitués par F, CN, SCN, SF₅, CH₂F, CHF₂, CF₃, OCH₂F, OCHF₂ ou OCF₃,
une ou plusieurs liaison(s) double(s) peut/peuvent être remplacée(s) par des liaisons simples, M, M¹ ou M² représente -O-, -S-, -CH₂-, -CHY- ou -CYY¹- de telle sorte que des groupes adjacents ne représentent pas simultanément -O- ou -S-, et
Y et Y¹ représentent Cl, F, CN, OCF₃ ou CF₃,
V représente H ou F,
Z¹, Z² et Z³ représentent, chacun indépendamment des autres, de manière identique ou différente, une liaison simple, -CH₂O-, -(CO)O-, -CF₂O-, -CH₂CH₂CF₂O-, -CF₂CF₂-, -CH₂CF₂-, -CH₂CH₂-, -(CH₂)₄-, -CH=CH-, -CH=CF-, -CF=CF- ou -C≡C-, où des ponts asymétriques peuvent être orientés sur les deux côtés, et
a représente 0, 1 ou 2,
b représente 0, 1 ou 2, et
c représente 0, 1 ou 2,
où a + b + c est ≤ 4.

2. Composés selon la revendication 1 de la formule IA: 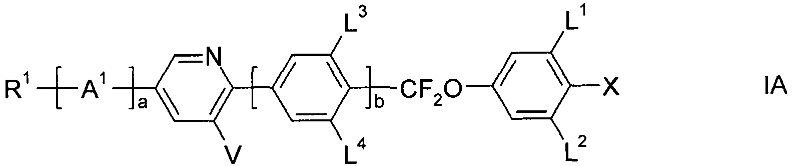 dans laquelle :
R¹, A¹, a, b et V présentent les significations indiquées pour la formule I selon la revendication 1,
X représente F, OCF₃, CN, CF₃, SCN, SF₅, NCS, Cl, OCHF₂, OCHFCF₃, OCF₂CHFCF₃,
V représente H ou F, et
L¹, L², L³ et L⁴ représentent, chacun indépendamment des autres, H ou F.

3. Composés selon la revendication 1 ou 2, **caractérisé en ce que** :
R¹ représente alkyle, alcoxy, alkényle ou alkényloxy comportant jusqu'à 8 atomes de carbone.

4. Composés selon une ou plusieurs des revendications 2 à 3, **caractérisés en ce que** L¹ représente fluor et L² représente, de manière indépendante, fluor ou hydrogène.

5. Composés selon une ou plusieurs des revendications 1 à 4 des formules I1 à 17 :
| | |
|---|---|
| 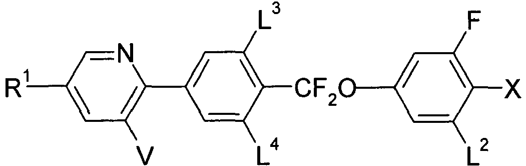 | I1 |
| 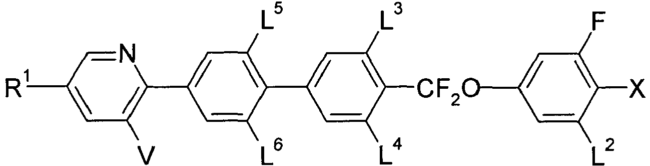 | I2 |
| 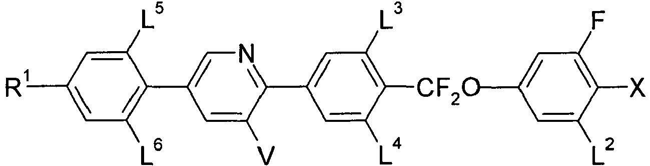 | I3 |
| 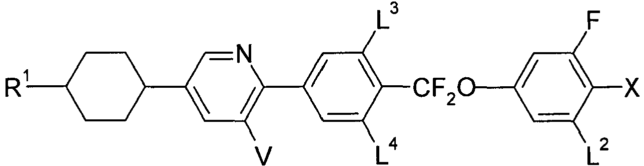 | I4 |
| 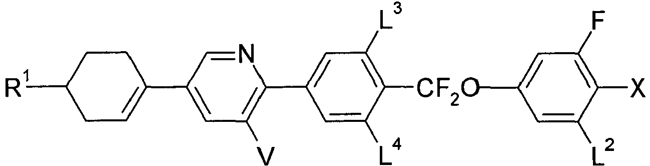 | I5 |
| 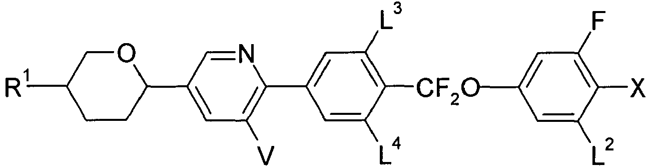 | I6 |
| 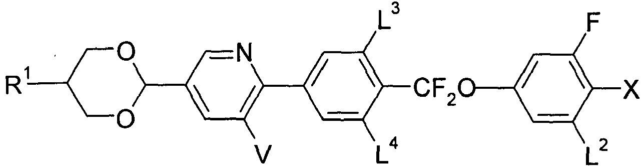 | I7 |
dans lesquelles R¹ et V présentent les significations indiquées selon la revendication 1, et
X représente F, OCF₃, CN, CF₃, SCN, SF₅, NCS, Cl, OCHF₂, OCHFCF₃, OCF₂CHFCF₃ et
L², L³, L⁴, L⁵ et L⁶ représentent H ou F.

6. Composés selon une ou plusieurs des revendications 2 à 5, **caractérisés en ce que** L¹ et L² représentent fluor.

7. Composés selon une ou plusieurs des revendications 1 à 6, **caractérisés en ce que** V représente hydrogène.

8. Composés selon une ou plusieurs des revendications 1 à 6, **caractérisés en ce que** V représente fluor.

9. Procédé pour la préparation de composés de la formule I selon une ou plusieurs des revendications 1 à 8, dans lequel V représente fluor ou hydrogène, **caractérisé en ce qu'**il comprend une étape de procédé selon laquelle un pyridine 2-substitué de la formule : 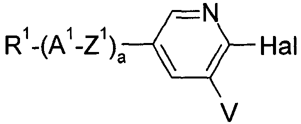 dans laquelle R¹, A¹, Z¹ et a sont comme défini selon la revendication 1, et
Hal représente OS0₂CF₃, CI, Br ou I,
est amené à réagir avec un acide boronique ou un ester d'acide boronique en chaîne ouverte ou cyclique des formules :
(OH)₂B-(Z²-A²)_{b}-CF₂O-(A³-Z³)-A⁴-R² 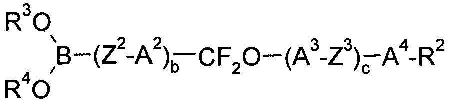
dans lesquelles Z², Z³, A², A³, A⁴, a, b et R² sont comme défini selon la revendication 1,
et
R³, R⁴ représentent alkyle comportant 1-12 atome(s) de C ou R³+R⁴ représentent également ensemble un C₂-C₈-alkylène ou un 1,2-phénylène,
où R³, R⁴ et R³+R⁴ peuvent également être substitués, en présence d'un catalyseur constitué par un métal de transition.

10. Utilisation d'un ou de plusieurs composé(s) de la formule I selon une ou plusieurs des revendications 1 à 8 en tant que composants dans un milieu cristallin liquide.

11. Milieu cristallin liquide comprenant au moins deux composés méso-gènes, **caractérisé en ce qu'**il comprend au moins un composé de la formule I selon une ou plusieurs des revendications 1 à 8.

12. Utilisation du milieu cristallin liquide selon la revendication 11 à des fins électro-optiques.

13. Affichage à cristaux liquides électro-optique contenant un milieu cristallin liquide selon la revendication 11.
